(19)

Europäisches
Patentamt

European
Patent Office

Office européen
des brevets

(11) **EP 4 751 698 A1**

(12) **EUROPEAN PATENT APPLICATION**
published in accordance with Art. 153(4) EPC

(43) Date of publication:
**03.06.2026 Bulletin 2026/23**

(21) Application number: **24845177.5**

(22) Date of filing: **27.05.2024**

(51) International Patent Classification (IPC):
*A61K 6/887* (2020.01)       *A61K 6/15* (2020.01)
*A61K 6/16* (2020.01)        *A61K 6/17* (2020.01)
*C08F 220/20* (2006.01)      *C08F 220/26* (2006.01)
*C08F 220/36* (2006.01)

(52) Cooperative Patent Classification (CPC):
**A61K 6/15; A61K 6/16; A61K 6/17; A61K 6/887;
C08F 220/20; C08F 220/26; C08F 220/36**

(86) International application number:
**PCT/JP2024/019347**

(87) International publication number:
**WO 2025/022795 (30.01.2025 Gazette 2025/05)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **26.07.2023 JP 2023121786**

(71) Applicant: **Tokuyama Dental Corporation
Tokyo 110-0016 (JP)**

(72) Inventors:
• **HANADA Ryu
Tokyo 110-0016 (JP)**
• **KIRA Ryuta
Tokyo 110-0016 (JP)**
• **MATSUO Takuma
Tokyo 110-0016 (JP)**

(74) Representative: **Gill Jennings & Every LLP
The Broadgate Tower
20 Primrose Street
London EC2A 2ES (GB)**

(54) **METHOD FOR PRODUCING RADIOPAQUE FILLER AND METHOD FOR PRODUCING DENTAL CURABLE COMPOSITION**

(57)   Provided is a method of efficiently producing an X-ray opaque filler, which can impart high X-ray opacity to a cured body of an existing structural color-based dental curable composition, without impairing the features of the composition, when blended into the composition. A slurry containing such a low crystalline rare earth metal fluoride particulate object that the full width at half maximum of the maximum intensity peak derived from the rare earth metal fluoride particulate object measured by an X-ray diffraction measurement method is 0.3° or more is subjected to spray drying to provide a particulate object. The particulate object and a polymerizable monomer are kneaded under a pressure of from 1.0 to $1.0 \times 10^5$ (Pa), and then the kneaded product is polymerized and cured. After that, pulverization and particle size adjustment are performed to produce an X-ray opaque filler formed of organic-inorganic composite particles in each of which the above-mentioned particulate object is dispersed in a resin.

EP 4 751 698 A1

## Description

Technical Field

The present invention relates to a method of producing an X-ray opaque filler and a method of producing a dental curable composition.

Background Art

In dental treatment, after the formation of a cavity in a carious tooth through the removal of a carious portion thereof, the following restoration (CR restoration) has been performed: the cavity is filled with a filling material called a composite resin (hereinafter also simply referred to as "CR"), which is formed of a dental curable composition including a polymerizable monomer, a filler, and a polymerization initiator as main components; and then the filling material is cured to seal the cavity. The above-mentioned CR restoration has the following features: the amount of a tooth substance to be removed by cutting can be reduced, and hence a color tone equivalent to a natural tooth color can be imparted; and its operation is easy. In addition, the CR restoration has achieved an improvement in mechanical strength of a CR cured body and an improvement in adhesive strength thereof with a tooth. Accordingly, the CR has been used not only in the restoration of anterior teeth but also in posterior teeth where a high occlusal pressure is applied.

To perform restoration having high aesthetics in the CR restoration, the following is generally performed: the color (hue and color tone) of a tooth to be restored (restoration target tooth) is determined (such color determination is sometimes referred to as "shade taking"); and a CR having a color compatible with the determined color is selected to perform the restoration. When such high-aesthetic restoration that a change in color between the sites of the tooth is faithfully reproduced is performed, there has been a need to perform the restoration through the lamination of a plurality of CRs having different colors. In addition, the coloring of a CR is typically performed by blending the CR with a pigment substance or a dye substance. In a cured body after its treatment, however, such substance undergoes color fading or discoloration owing to its deterioration over time. Accordingly, the following has occurred in some cases: as a time period elapses from the completion of the restoration, the color of the cured body changes, and hence the appearance of a restored site becomes no longer compatible with a natural tooth.

In recent years, the following CR has been proposed and attracting attention (see Patent Literatures 1 and 2): a filler containing spherical inorganic particles having a specific average particle diameter and a specific particle size distribution is used; and the refractive index of the spherical inorganic particles is made larger than the refractive index of a resin portion serving as a matrix at the time of the curing of the CR to enable the CR to express a structural color, which develops a predetermined color tone independent of the incidence angle of light, through the interference, scattering, or the like of the light (a CR expressing such structural color is hereinafter also referred to as "structural color-based CR").

The above-mentioned structural color-based CR has the following excellent features: (1) the problem of discoloration over time hardly occurs because the CR does not use any dye substance or pigment substance; (2) the CR expresses a structural color having a specific color tone, which is independent of the incidence angle of light, in accordance with the average particle diameter of the spherical inorganic particles to be used, and particularly when spherical inorganic particles having an average primary particle diameter of from 230 nm to 350 nm are used, the CR can be colored to from a yellow color to a red color serving as a color similar to a dentin color; and (3) a cured body of the CR has moderate transparency and hence easily harmonizes with the color of a restoration target tooth, and thus the restoration target teeth having a wide variety of colors can each be restored so as to have an appearance close to that of a natural tooth with one kind of composite resin without performance of complicated shade taking or the selection of the shade of the composite resin.

It has been known that when a polymerization-curable composition for forming the above-mentioned structural color-based CR, which satisfies the following conditions (a) and (b), further satisfies the following condition (c), the composition provides a cured body that expresses an expected structural color more reliably (see Patent Literature 2).

(a) The composition includes, as constituent components, a polymerizable monomer (A), inorganic particles (B), and a photopolymerization initiator (C).
(b) The inorganic particles (B) satisfy all of the following conditions:

(b-1) a condition that the particles include one or a plurality of "groups of spherical particles having identical diameters" (G-PIDs), which are each formed of an aggregate of inorganic spherical particles having a predetermined average primary particle diameter in the range of from 100 nm to 1,000 nm, and in each of which the individual inorganic spherical particles for forming the aggregate include substantially the same substance, and 90% or more of the total number of the particles are present in the range of ±5% of the predetermined average primary particle diameter in the number-based particle size distribution of the aggregate;

(b-2) a condition that when the number of the one or the plurality of "groups of spherical particles having identical diameters" is represented by "a", and the respective "groups of spherical particles having identical diameters" are each represented by G-PID$_m$ in order of increasing average primary particle diameter (provided that when "a" represents 1, "m" represents 1, and when "a" represents 2 or more, "m" represents a natural number of from 1 to "a"), the substances for forming the individual particles of the respective G-PID$_m$s in the case where the "a" represents 2 or more may be different from each other, and the average primary particle diameters of the respective G-PID$_m$s in the case are different from each other by 25 nm or more; and

(b-3) a condition that when the refractive index of a cured body of the polymerizable monomer component (A) at 25°C is represented by $n_{(MX)}$, and the refractive indices of the inorganic spherical particles for forming the respective G-PID$_m$s at 25°C are each represented by $n_{(G\text{-}PIDm)}$, the following relationship is valid for any one of the $n_{(G\text{-}PIDm)}$s.

$$n_{(MX)} < n_{(G\text{-}PIDm)}$$

(c) With regard to a cured body obtained by curing a polymerization-curable composition, when a radial distribution function g(r), which represents a probability that any other inorganic spherical particle is present at a point distant from the center of an arbitrary inorganic spherical particle dispersed in the cured body by a distance "r", is represented by

$$equation: \ g(r) = \{1/<\rho>\} \times \{dn/da\}$$

on the basis of the average particle density $<\rho>$ of the inorganic spherical particles in a plane inside the cured body serving as an observation plane, the number dn of the inorganic spherical particles present in a region between a circle distant from the arbitrary inorganic spherical particle in the observation plane by the "r" and a circle distant therefrom by r+dr, and the area da of the region (provided that da=$2\pi r \cdot dr$), the density, the number, and the area being each determined on the basis of a scanning electron microscope image of the observation plane, the dispersed state of the inorganic particles (B) in the cured body satisfies the following conditions (I) and (II).

[Conditions that Dispersed State should satisfy]

(I) In a radial distribution function graph showing a relationship between a normalized dimensionless number (r/r$_0$), which is obtained by dividing the distance "r" from the center of the arbitrary inorganic spherical particle dispersed in the cured body by the average particle diameter r$_0$ of the entirety of the inorganic spherical particles dispersed in the cured body, and the radial distribution function g(r) corresponding to the "r" at the time, the graph having an x-axis indicating the ratio "r/r$_0$" and a y-axis indicating the g(r), a nearest-neighbor particle distance r$_1$ defined as the "r" corresponding to the peak top of a peak that is closest to an origin out of peaks appearing in the radial distribution function graph is a value once or more and twice or less as large as the average particle diameter r$_0$ of the entirety of the inorganic spherical particles dispersed in the cured body of the mixture.

(II) When the "r" corresponding to the peak top of a peak that is second closest to the origin out of the peaks appearing in the radial distribution function graph is defined as a second-nearest-neighbor particle distance r$_2$, the local minimum value of the radial distribution function g(r) between the nearest-neighbor particle distance r$_1$ and the second-nearest-neighbor particle distance r$_2$ is a value of 0.56 or more and 1.10 or less.

According to Patent Literature 2, it has been conceived that colored light caused by interference in the cured body occurs in a portion whose constituent particles are integrated in a relatively regular manner, and colored light caused by scattering occurs in a portion whose constituent particles are dispersed disorderly. In addition, the following description has been made for the above-mentioned condition (I): when the r$_1$ is less than once as large as the r$_0$, the frequency at which the particles overlap each other in a plane increases; and when the r$_1$ is more than twice as large as the r$_0$, no particles are present near the selected central inorganic particle, and hence the short-distance orderliness of the array structure of the inorganic spherical particles is lost, with the result that no structural color is expressed. The following description has been made for the above-mentioned condition (II): when the local minimum value becomes less than 0.56, the long-distance orderliness of the array structure of the inorganic spherical particles is raised, and hence the dependence of the structural color to be expressed on the incidence angle of light is raised; moreover, the chroma of the cured body increases, and hence color tone compatibility in the case where the cured body is used as a dental filling material is hardly obtained; and meanwhile, when the above-mentioned local minimum value is more than 1.10, the array structure of the inorganic spherical particles becomes a random structure, and hence targeted reflection performance is hardly obtained, with the result that the expected structural color is hardly expressed.

In the polymerization-curable composition satisfying those conditions (a) to (c) (hereinafter also referred to as "existing structural color-based dental curable composition"), when the composition includes the plurality of groups of spherical particles having identical diameters (G-PIDs), an entire developed color tone can be controlled by the combination of the G-PIDs to be blended because each of the G-PIDs develops a structural color having a color tone in accordance with its average primary particle diameter in the cured body. A possible reason for the foregoing is as follows: when the composition includes the plurality of G-PIDs, and there is a certain difference between the sizes of their average primary particle diameters, the inorganic spherical particles belonging to the different G-PIDs can be dispersed while having a short-distance ordered structure, which can express a structural color for each G-PID, without replacing each other.

Incidentally, an inorganic oxide filler, in particular, a silica-based filler has been generally used as a filler to be blended into a curable composition to be used as a CR including the existing structural color-based dental curable composition. However, the silica-based filler has low X-ray opacity. Accordingly, at the time of radiography or computed tomography (CT) in the dental treatment, a cured product in the cavity is not imaged, and hence it is difficult to determine a treatment site.

Meanwhile, as means for improving the X-ray opacity of the cured body of a curable composition to be used in various applications, such as a curable composition to be used in the dental applications listed above, there has been known a method using a filler formed of a fluoride of a rare earth metal having an atomic number of from 57 to 71 (see Patent Literatures 3 and 4). The transparency of the cured body typically reduces with increasing content of the rare earth metal fluoride (see Patent Literature 3). However, when an "X-ray opaque filler characterized by being formed of any powder selected from the group consisting of: first powder, which contains crystalline rare earth metal fluoride particles as a main component, and in which the full width at half maximum of the maximum intensity peak derived from the crystalline rare earth metal fluoride particles in an X-ray diffraction pattern is 0.3° or more; and second powder obtained by treating the surface of the first powder" is used, the X-ray opacity of the cured body can be improved without any reduction in transparency of the cured body (see Patent Literature 4). According to Patent Literature 4, "such a particulate object formed of crystalline rare earth metal fluoride particles that the full width at half maximum of the maximum intensity peak in an X-ray diffraction pattern obtained when the particulate object is subjected to X-ray diffraction measurement is 0.3° or more" (hereinafter also referred to as "low crystalline rare earth metal fluoride particulate object"), the particulate object serving as the above-mentioned first powder, is obtained by subjecting such a particulate object that the full width at half maximum is less than 0.3° (hereinafter also referred to as "highly crystalline rare earth metal fluoride particulate object") to mechanochemical treatment. In addition, the full width at half maximum of the low crystalline rare earth metal fluoride particulate object is preferably 40° or less.

Citation List

Patent Literature

[PTL 1] WO 2017/069274 A1
[PTL 2] WO 2020/050123 A1
[PTL 3] JP 03-17803 B2
[PTL 4] WO 2023/042598 A1

Summary of Invention

Technical Problem

However, an investigation made by the inventors of the present invention has recognized that when such an X-ray opaque filler as disclosed in Patent Literature 4 described above is blended as an X-ray opaque filler into an existing structural color-based dental curable composition, the above-mentioned reduction in transparency is suppressed, but the expression of an expected structural color may be adversely affected (see Reference Comparative Examples 2 to 4 to be described later).

The present invention has been made in view of the above-mentioned circumstances, and an object of the present invention is to provide a method of efficiently producing the following X-ray opaque filler: when the X-ray opaque filler is blended into an existing structural color-based dental curable composition (suitable as a structural color-based CR capable of aesthetic restoration), the filler can impart high X-ray opacity to a cured body thereof without impairing the features of the composition.

Solution to Problem

The present invention is intended to solve the above-mentioned problems, and a first aspect of the present invention is a method of producing an X-ray opaque filler, which is configured to impart X-ray opacity to each of a dental curable composition containing a polymerizable monomer and a cured body thereof by being blended into the dental curable composition, wherein

when such a particulate object formed of crystalline rare earth metal fluoride particles that a full width at half maximum of a maximum intensity peak in an X-ray diffraction pattern obtained when the particulate object is subjected to X-ray diffraction measurement is less than 0.3° is defined as "highly crystalline rare earth metal fluoride particulate object," and such a particulate object that the full width at half maximum is 0.3° or more is defined as "low crystalline rare earth metal fluoride particulate object,"
the X-ray opaque filler contains, as a main component, a particulate object, which includes organic-inorganic composite particles each formed of a composite material obtained by dispersing the low crystalline rare earth metal fluoride particulate object in a resin matrix, and which has an average particle diameter measured by a laser diffraction/scattering method of from 3 $\mu$m to 110 $\mu$m,
the method including:

a secondary raw material particulate object-preparing step of subjecting a raw material slurry, which is obtained by dispersing a primary raw material particulate object formed of a highly crystalline rare earth metal fluoride particulate object having an average primary particle diameter measured by electron microscope observation of from 1 nm to 500 nm in a dispersion medium, to mechanochemical treatment to provide a treated slurry liquid in which the low crystalline rare earth metal fluoride particulate object is dispersed in the dispersion medium, followed by spray drying of the treated slurry thus obtained to provide a secondary raw material particulate object formed of the low crystalline rare earth metal fluoride particulate object;
a curable raw material composition-preparing step of kneading 100 parts by mass of the polymerizable monomer serving as a raw material for the resin matrix and 150 parts by mass to 900 parts by mass of the secondary raw material particulate object under a pressure of from 1.0 to $1.0 \times 10^5$ (Pa) to provide a curable raw material composition;
a curing step of curing the curable raw material composition to provide a bulk material formed of a composite material obtained by dispersing low crystalline rare earth metal fluoride particles in the resin matrix; and
a granulation step of pulverizing the bulk material, followed by particle size adjustment so that the average particle diameter becomes from 3 $\mu$m to 110 $\mu$m.

In the production method of the above-mentioned aspect (hereinafter also referred to as "the present X-ray opaque filler production method"), the amount of the secondary raw material particulate object to be used in the curable raw material composition-preparing step is preferably from 230 parts by mass to 900 parts by mass with respect to 100 parts by mass of the polymerizable monomer.

In addition, the pressure at the time of the kneading in the curable raw material composition-preparing step is preferably from 100 to 35,000 (Pa).

A second aspect of the present invention is a method of producing a dental curable composition, which is capable of providing a cured body that expresses a structural color having a predetermined color tone independent of an incident angle of light and has X-ray radiography contrast, by mixing:

100 parts by mass of a polymerizable monomer;
1 part by mass to 100 parts by mass of an X-ray opaque filler containing, as a main component, a particulate object, which includes organic-inorganic composite particles each formed of a composite material obtained by dispersing a low crystalline rare earth metal fluoride particulate object in a resin matrix, and which has an average particle diameter measured by a laser diffraction/scattering method of from 3 $\mu$m to 110 $\mu$m, in terms of a total mass of the low crystalline rare earth metal fluoride particulate object;
10 parts by mass to 1,500 parts by mass in total of one or a plurality of "groups of spherical particles having identical diameters," which are each formed of an aggregate of inorganic spherical particles having a predetermined average primary particle diameter measured by electron microscope observation in a range of from 100 nm to 1,000 nm, the individual inorganic spherical particles for forming the aggregate including substantially the same substance, and 90% or more of a total number of the particles being present in a range of $\pm 5\%$ of the predetermined average primary particle diameter in a number-based particle size distribution of the aggregate; and

0.01 part by mass to 10 parts by mass of a polymerization initiator,

the method further including, as a step of preparing the X-ray opaque filler, a step of producing the X-ray opaque filler by the present X-ray opaque filler production method,

wherein in the step, the X-ray opaque filler is produced by using a polymerizable monomer serving as a raw material for the resin matrix, the polymerizable monomer satisfying the following condition: when a refractive index of a cured body of the polymerizable monomer for a sodium d-line at 25°C is represented by $n_{(MX)}$, and a refractive index of a resin material for forming the resin matrix of each of the organic-inorganic composite particles for forming the X-ray opaque filler for the sodium d-line at refractive index 25°C is represented by $n_{(F-MX)}$, an absolute value $|n_{(MX)}-n_{(F-MX)}|$ of a difference between the $n_{(MX)}$ of the polymerizable monomer to be actually mixed and the $n_{(F-MX)}$ is from 0 to 0.1.

In the method of producing a dental curable composition of the above-mentioned aspect (hereinafter also referred to as "the present dental curable composition production method"), it is preferred that when the number of the groups of spherical particles having identical diameters" in the one or the plurality of "groups of spherical particles having identical diameters" is represented by "a", the respective "groups of spherical particles having identical diameters" are each represented by G-PID$_m$ in order of increasing average primary particle diameter, provided that "m" represents 1 when "a" represents 1, and "m" represents a natural number of from 1 to "a" when "a" represents 2 or more, and a refractive index of the inorganic spherical particles for forming each of the G-PID$_m$s for the sodium d-line at 25°C is represented by $n_{(G-PIDm)}$,

the substances for forming the individual particles of the respective G-PID$_m$s in a case where the "a" represents 2 or more may be different from each other,

the average primary particle diameters of the respective G-PID$_m$s in the case be different from each other by 25 nm or more, and

a relationship of $n_{(MX)} < n_{(G-PIDm)}$ be valid for any one of the $n_{(G-PIDm)}$s.

Advantageous Effects of Invention

According to the present invention, there can be efficiently produced a novel X-ray opaque filler, which can impart high X-ray opacity to a cured body of an existing structural color-based dental curable composition, without impairing the excellent features of the structural color-based CR, by being blended into the composition.

Description of Embodiments

The X-ray opaque filler (low crystalline rare earth metal fluoride particulate object) disclosed in Patent Literature 4 has such excellent features as described above. However, when the inventors of the present invention have blended the X-ray opaque filler into a structural color-based CR, the inventors have found that a large amount of the X-ray opaque filler needs to be blended in order to obtain high X-ray opacity, and in that case, the expression of an expected structural color becomes difficult (see Reference Comparative Examples 3 and 4 to be described later). In view of the foregoing, as a result of a further investigation to solve such problem, the inventors have found that: when the low crystalline rare earth metal fluoride particulate object is blended not directly but as a particulate object, which is formed of organic-inorganic composite particles obtained by compositing the low crystalline rare earth metal fluoride particulate object with an organic resin, into a curable composition, even in the case where the blending amount of rare earth metal fluoride particles is small, high X-ray opacity can be imparted to a cured body of the curable composition; and moreover, even in the case where the particulate object is blended into a structural color CR, X-ray opacity can be imparted to a cured body thereof without any significant adverse effect on the expression of the expected structural color (see Reference Examples to be described later). The foregoing has already been proposed (Japanese Patent Application No. 2023-29645).

That is, as a dental curable composition, which can impart high X-ray opacity to a cured body of the composition without impairing the excellent features of the structural color-based CR when the composition is used as a CR, there has been proposed a "dental curable composition, including: 100 parts by mass of a polymerizable monomer; 10 parts by mass to 1,500 parts by mass in total of one or a plurality of "groups of spherical particles having identical diameters" (G-PIDs), which are each formed of an aggregate of inorganic spherical particles having a predetermined average primary particle diameter in a range of from 100 nm to 1,000 nm, and in each of which the individual inorganic spherical particles for forming the aggregate include substantially the same substance, and 90% or more of the total number of the particles are present in a range of ±5% of the predetermined average primary particle diameter in a number-based particle size distribution of the aggregate; and 0.01 part by mass to 0.5 part by mass of a polymerization initiator,

in which when the number of the one or the plurality of "groups of spherical particles having identical diameters" is represented by "a", and the respective "groups of spherical particles having identical diameters" are each represented by $G\text{-PID}_m$ in order of increasing average primary particle diameter, provided that when "a" represents 1, "m" represents 1, and when "a" represents 2 or more, "m" represents a natural number of from 1 to "a", the substances for forming the individual particles of the respective $G\text{-PID}_m$s in a case where the "a" represents 2 or more may be different from each other, and the average primary particle diameters of the respective $G\text{-PID}_m$s in the case are different from each other by 25 nm or more, and

in which when a refractive index of a cured body of the polymerizable monomer for a sodium d-line at 25°C is represented by $n_{(MX)}$, and refractive indices of the inorganic spherical particles for forming the respective $G\text{-PID}_m$s for the sodium d-line at 25°C are each represented by $n_{(G\text{-PIDm})}$, the following relationship is valid for any one of the $n_{(G\text{-PIDm})}$s:

$$n_{(MX)} < n_{(G\text{-PIDm})},$$

the dental curable composition being capable of providing a cured body that expresses a structural color having a predetermined color tone independent of an incidence angle of light,

wherein the dental curable composition includes 1 part by mass to 100 parts by mass of an X-ray opaque filler (hereinafter also referred to as "specific X-ray opaque filler"), including organic-inorganic composite particles in each of which when crystallinity of each of crystalline rare earth metal fluoride particles for forming powder formed of the crystalline rare earth metal fluoride particles is represented by a full width at half maximum (unit: °) of a maximum peak derived from the crystalline rare earth metal fluoride in an X-ray diffraction pattern obtained when the powder is subjected to X-ray diffraction measurement, a plurality of such crystalline rare earth metal fluoride particles that the full width at half maximum is 0.3° or more are dispersed in a resin matrix, in terms of a total mass of the crystalline rare earth metal fluoride particles, and

wherein an absolute value $|n_{(MX)}-n_{(F\text{-MX})}|$ of a difference between the refractive index $n_{(MX)}$ of the cured body of the polymerizable monomer for the sodium d-line at 25°C and a refractive index $n_{(F\text{-MX})}$ of a resin material for forming the resin matrix of each of the organic-inorganic composite particles for forming the X-ray opaque filler for the sodium d-line at 25°C is from 0 to 0.1."

The above-mentioned specific X-ray opaque filler can be said to be an X-ray opaque filler that can impart high X-ray opacity to a cured body of an existing structural color-based dental curable composition without impairing the features of the composition when blended into the composition so as to satisfy the following condition: the above-mentioned absolute value of the difference $|n_{(MX)}-n_{(F\text{-MX})}|$ is from 0 to 0.1. However, in Japanese Patent Application No. 2023-29645, as described in Reference Examples to be described later, the specific X-ray opaque filler has been obtained (on a laboratory scale) by: kneading a particulate object (secondary raw material particulate object), which is obtained by drying a treated liquid (treated slurry) having dispersed therein a low crystalline rare earth metal fluoride particulate object obtained after mechanochemical treatment with a rotary evaporator, and a polymerizable monomer with an agate mortar; and pulverizing a cured product obtained by curing the resultant curable composition. Accordingly, it cannot be said that a method of efficiently producing the specific X-ray opaque filler on an industrial scale is established.

In view of the foregoing, the inventors of the present invention have attempted to prepare a curable composition by: obtaining the secondary raw material particulate object through spray drying with a spray dryer or the like; and then kneading the particulate object and the polymerizable monomer with a kneading apparatus. As a result, it has become clear that when the content of the low crystalline rare earth metal fluoride particulate object in the organic-inorganic composite particles for forming the specific X-ray opaque filler is increased to impart X-ray opacity to a cured body of the composition, there arises a problem in that the kneaded product does not become pasty, and hence the kneading sometimes becomes impossible.

The present invention is intended to solve the above-mentioned problem that is peculiar to a case in which spray drying with a spray dryer and kneading with a kneading apparatus are performed as described above. The present invention enables efficient production of the specific X-ray opaque filler even in the above-mentioned case through the performance of the above-mentioned kneading (mixing) under a specific reduced pressure.

The specific X-ray opaque filler is a novel material found by the inventors of the present invention, and hence the specific X-ray opaque filler is described first, and then the present X-ray opaque filler production method and the like are described in detail below.

The expression "from "x" to "y"" using numerical values "x" and "y" as used herein means ""x" or more and "y" or less" unless otherwise specified. When a unit is assigned only to the numerical value "y" in such expression, the unit is applied to the numerical value "x" as well. In addition, the term "(meth)acrylic" as used herein means both of "acrylic" and "methacrylic". Similarly, the term "(meth)acrylate" means both of "acrylate" and "methacrylate", and the term "(meth)acryloyl" means both of "acryloyl" and "methacryloyl". Further, the term "(meth)acrylic resin" means <i> a polymer polymerized by using only a (meth)acrylate-based monomer as a polymerizable monomer to be used in the polymerization of the (meth)acrylic resin, or <ii> such a polymer that when two or more kinds of polymerizable monomers including the (meth)acrylate-based monomer are used, the ratio of the (meth)acrylate-based monomer to all the polymerizable monomers is 50 mol% or more.

1. Specific X-ray Opaque Filler

(1) Overview of Specific X-ray Opaque Filler

The specific X-ray opaque filler contains, as its main component, a particulate object, the particulate object including organic-inorganic composite particles in each of which a plurality of such crystalline rare earth metal fluoride particles as described below are dispersed in a resin matrix, the particulate object having an average particle diameter measured by a laser diffraction/scattering method of from 3 $\mu$m to 110 $\mu$m: when the crystallinity of individual crystalline rare earth metal fluoride particles for forming a particulate object formed of the crystalline rare earth metal fluoride particles is represented by the full width at half maximum (unit: °) of the maximum peak derived from the crystalline rare earth metal fluoride in an X-ray diffraction pattern obtained when the particulate object is subjected to X-ray diffraction measurement, the full width at half maximum is 0.3° or more.

Here, the phrase "contains, as its main component" means that the content of the organic-inorganic composite particles (hereinafter also referred to as "particle body") in the total mass of the specific X-ray opaque filler is 95 mass% or more, preferably 98 mass% or more. A component that the specific X-ray opaque filler may contain in addition to the particle body may be, for example, external additive particles, such as silica or titanium oxide (fine particles). In addition, the surface of each of the organic-inorganic composite particles (particle body) may be subjected to physical surface treatment such as plasma treatment or to mechanical surface treatment by, for example, long-term friction and stirring. Alternatively, the surface may be subjected to coating agent treatment with a known coating agent such as a silicone oil. Further, the crystalline rare earth metal fluoride particles in the organic-inorganic composite particles (particle body) may each be subjected to surface treatment with a known surface treatment agent, such as a silane coupling agent or a titanate coupling agent.

It has been generally known that there is a correlation known as a Scherrer equation between the full width at half maximum of a diffraction peak in X-ray diffraction measurement and a crystallite size, and the crystallite size is inversely proportional to the full width at half maximum. In addition, the full width at half maximum (FWHM) is affected by the strain of a crystal lattice so that the full width at half maximum (FWHM) may be larger. The full width at half maximum tends to widen as the crystal lattice strain becomes larger. It can be said that the full width at half maximum is an indicator of the crystallinity (more specifically, crystal perfection) of a rare earth metal fluoride because the crystallinity may reduce when fine crystallites are oriented toward various directions through the enlargement of a crystallite strain and a reduction in crystallite diameter.

Unless otherwise stated, the term "full width at half maximum" as used herein below means the full width at half maximum (unit: °) of the maximum peak derived from the crystalline rare earth metal fluoride in the X-ray diffraction pattern obtained when the particulate object formed of the crystalline rare earth metal fluoride particles is subjected to the X-ray diffraction measurement.

The crystalline rare earth metal fluoride particulate object having a full width at half maximum of 0.3° or more (low crystalline rare earth metal fluoride particulate object) does not reduce the transparency of a cured body of the dental curable composition to a very large extent even when the particulate object is blended as it is into the dental curable composition (see PCT/JP2022/031237, and Reference Comparative Examples 3 and 4 to be described later). The reason for the expression of such effect is presumed to be as described below. First, in a system in which inorganic fine particles are dispersed in a resin matrix, a reduction in transparency is largely affected by diffuse reflection of light at an interface between each of the particles and the resin matrix. Meanwhile, it is conceived that, in each of the crystalline rare earth metal fluoride particles, a vicinity of the surface thereof is gradually amorphized from the surface to the inside thereof through the mechanochemical treatment. In addition, when such phenomenon occurs, a layer whose refractive index is gradually reduced at a certain gradient from the inside to the surface (hereinafter also referred to as "refractive index gradient layer") is formed in the vicinity of the surface of each of the crystalline rare earth metal fluoride particles. Moreover, the refractive index gradient layer formed includes a portion having a refractive index consistent with the refractive index of the resin matrix. As a result, it is conceived that the ratio of reflected light is reduced (the ratio of transmitted light is increased) as a whole, and the reduction in transparency is suppressed.

Even when the specific X-ray opaque filler containing the organic-inorganic composite particles (particle body) as its main component is used, the same effect is obtained in the case where a polymerizable monomer of the dental curable composition satisfies the following condition: the absolute value $|n_{(MX)}-n_{(F-MX)}|$ of a difference between the refractive index $n_{(MX)}$ of a cured body of the polymerizable monomer for a sodium d-line at 25°C and the refractive index $n_{(F-MX)}$ of a resin material for forming the resin matrix of each of the organic-inorganic composite particles for forming the specific X-ray opaque filler for the sodium d-line at refractive index 25°C is from 0 to 0.1, preferably from 0 to 0.05. A possible reason for the foregoing is as described below. Although the polymerizable monomer in the dental curable composition serves as a resin for forming the matrix of the cured body of the dental polymerizable composition, when the value of its refractive index $n_{(F-MX)}$ and the value of the refractive index $n_{(F-MX)}$ of the resin material for forming the resin matrix of each of the organic-inorganic composite particles are approximate to each other, the diffuse reflection of light hardly occurs on the surface of each of the organic-inorganic composite particles (in other words, the resin matrix of each of the organic-inorganic composite particles and the resin matrix of the cured body of the dental curable composition are integrated with each other). Thus, the above-mentioned characteristic of the low crystalline rare earth metal fluoride particulate object is exhibited.

As described above, when the full width at half maximum of the crystalline rare earth metal fluoride particulate object is set to 0.3° or more, the curing of the curable composition blended with the specific X-ray opaque filler can easily provide a cured body excellent in both of X-ray opacity and transparency. That is, even when the blending amount of the specific X-ray opaque filler to be blended into the curable composition is increased to improve the X-ray opacity of the cured body, a reduction in transparency of the cured body can be suppressed. The full width at half maximum only needs to be 0.3° or more, and is preferably 0.4° or more, more preferably 0.5° or more. Meanwhile, the upper limit value of the full width at half maximum is not particularly limited. In practical use, however, the upper limit value is preferably 40° or less, more preferably 1° or less.

When a crystalline rare earth metal fluoride particulate object having a full width at half maximum of less than 0.3° (highly crystalline rare earth metal fluoride particulate object) is blended in an amount required to impart X-ray opacity without being changed or after having been subjected to organic-inorganic composition (after having been turned into the same organic-inorganic composite particles as the organic-inorganic composite particles in the specific X-ray opaque filler), a reduction in transparency of the cured body is inevitable (see Reference Comparative Examples 2 and 5 to be described later).

(2) Method of determining Full Width at Half Maximum

The full width at half maximum may be determined by subjecting a powder sample serving as an object of X-ray diffraction measurement to the X-ray diffraction measurement. Specifically, the performance of the X-ray diffraction measurement of the powder sample with an X-ray diffractometer in the $2\theta$ range of from 20° to 120° provides an X-ray diffraction pattern (chart) whose axis of abscissa indicates $2\theta$ (°), and whose axis of ordinate indicates a diffraction intensity. A sample from which coarse particles have been removed in accordance with an ordinary method by, for example, using a sieve having an aperture of 100 $\mu$m is preferably utilized as the powder sample.

Next, peaks derived from the rare earth metal fluoride in the X-ray diffraction pattern (chart) are identified, and the full width at half maximum of a peak having the maximum intensity out of the plurality of peaks thus identified is determined. Description is given by taking, as a specific example, a case in which the rare earth metal fluoride is $YbF_3$. The peak having the maximum intensity appears as a peak resulting from a (111) plane at a $2\theta$ of about 28.0°. Here, the full width at half maximum is obtained by determining, in the peak observed at a $2\theta$ of about 28.0°, a peak width at an intensity corresponding to 50% of the local maximum intensity of the peak (50% intensity). The peak width is determined as follows: two points of intersection at which a peak line having a convex shape and a straight line, which is parallel to the axis of abscissa of the X-ray diffraction pattern (chart) and is positioned at the 50% intensity, intersect each other are identified; and the absolute value (unit: "deg [°]") of a difference between the value of the $2\theta$ at one of the points of intersection and the value of the $2\theta$ at the other point of intersection is determined as the width.

The full width at half maximum of the crystalline rare earth metal fluoride particulate object in the organic-inorganic composite particles (particle body) for forming the specific X-ray opaque filler may be identified on the basis of an X-ray diffraction pattern obtained by subjecting the powder sample formed of the particle body to powder X-ray diffraction measurement. In addition, with regard to a specific X-ray opaque filler obtained by a production method to be described later, the full width at half maximum of the crystalline rare earth metal fluoride particulate object in the particle body may be identified on the basis of an X-ray diffraction pattern obtained by subjecting a powder sample formed of a secondary raw material particulate object to the powder X-ray diffraction measurement (because the crystallinity of the secondary raw material particulate object remains unchanged or substantially unchanged in a mixing or pulverizing step at the time of the preparation of a raw material composition). Further, the full width at half maximum of the crystalline rare earth metal fluoride particles in the specific X-ray opaque filler in the dental curable composition of the present invention may be identified on the basis of an X-ray diffraction pattern obtained by subjecting a powder sample, which contains the organic-inorganic composite particles separated from such curable composition, or a powder sample obtained from the cured body of such curable composition to the powder X-ray diffraction measurement.

(3) Organic-Inorganic Composite Particles (Particle Body)

A material for the rare earth metal fluoride particulate object in the organic-inorganic composite particles (particle body) is not particularly limited as long as the rare earth metal fluoride particulate object has a full width at half maximum of 0.3° or more, and known rare earth metal fluoride particles may be appropriately used. In addition, the rare earth metal fluoride is preferably lanthanum fluoride ($LaF_3$), cerium fluoride ($CeF_3$), or ytterbium fluoride ($YbF_3$) from the viewpoint that a color tone and safety suitable for the dental applications are easily secured. Further, the rare earth metal fluoride is particularly preferably ytterbium fluoride ($YbF_3$) from the viewpoint of securing X-ray opacity.

The resin material for forming the resin matrix of each of the organic-inorganic composite particles (particle body) is not particularly limited, and examples thereof may include a (meth)acrylic resin and a polyaryl ether ketone resin.

However, when the specific X-ray opaque filler is blended into an existing structural color-based dental curable composition, in order that the structural color-expressing effect of the composition may not be impaired, such a resin material that the absolute value $|n_{(MX)}-n_{(F-MX)}|$ is from 0 to 0.1 needs to be used as a resin material for forming the resin matrix. Herein, the $n_{(MX)}$ means the refractive index of a cured body of a polymerizable monomer in the existing structural color-based dental curable composition for a sodium d-line at 25°C, and the $n_{(F-MX)}$ means the refractive index of the resin material for forming the resin matrix of the particle body for the sodium d-line at 25°C.

The average particle diameter of the organic-inorganic composite particles (particle body) is from 3 $\mu$m to 110 $\mu$m, preferably from 8 $\mu$m to 100 $\mu$m. Herein, the above-mentioned average particle diameter means a median diameter measured by a laser diffraction/scattering method. From the viewpoint that both the X-ray radiography contrast and mechanical strength of a cured body of the curable composition can be achieved in a more balanced manner, when the average particle diameter deviates from the above-mentioned ranges, the above-mentioned average particle diameter is more preferably from 22 $\mu$m to 70 $\mu$m, in particular, from 24 $\mu$m to 55 $\mu$m. In addition, when higher emphasis is placed on the securement of the mechanical strength than the X-ray radiography contrast, the average particle diameter is preferably from 3 $\mu$m to 38 $\mu$m, more preferably from 8 $\mu$m to 25 $\mu$m. In contrast, when higher emphasis is placed on the securement of the X-ray radiography contrast than the mechanical strength, the average particle diameter is preferably 38 $\mu$m or more, more preferably 70 $\mu$m or more. The upper limit value is preferably 110 $\mu$m or less, more preferably 100 $\mu$m or less, from the viewpoint of securing a certain level or more of mechanical strength.

In addition, the content of the rare earth metal fluoride particles to be incorporated into the organic-inorganic composite particles (particle body) is preferably from 60 mass% to 90 mass%, more preferably from 70 mass% to 80 mass% with respect to the total mass of the organic-inorganic composite particles.

2. The Present X-Ray Opaque Filler Production Method

The present X-ray opaque filler production method is characterized by including:

a secondary raw material particulate object-preparing step of subjecting a raw material slurry, which is obtained by dispersing a primary raw material particulate object formed of a highly crystalline rare earth metal fluoride particulate object having an average primary particle diameter measured by electron microscope observation of from 1 nm to 500 nm in a dispersion medium, to mechanochemical treatment to provide a treated slurry liquid in which a low crystalline rare earth metal fluoride particulate object is dispersed in the dispersion medium, followed by the removal of the dispersion medium of the treated slurry thus obtained to provide a secondary raw material particulate object formed of a low crystalline rare earth metal fluoride particulate object;

a curable raw material composition-preparing step of kneading 100 parts by mass of a polymerizable monomer and 230 parts by mass to 900 parts by mass of the secondary raw material particulate object under a pressure of from 1.0 to $1.0\times10^5$ (Pa) (under reduced pressure) in terms of absolute pressure to provide a curable raw material composition;

a curing step of curing the curable raw material composition to provide a bulk material formed of a composite material obtained by dispersing low crystalline rare earth metal fluoride particles in a resin matrix; and

a granulation step of pulverizing the bulk material, followed by particle size adjustment so that the average particle diameter becomes from 3 $\mu$m to 110 $\mu$m.

Each step is described in detail below.

(1) Secondary Raw Material Particulate Object-preparing Step

In the secondary raw material particulate object-preparing step, the secondary raw material particulate object formed of the low crystalline rare earth metal fluoride particulate object is obtained by: subjecting the raw material slurry, which is obtained by dispersing the primary raw material particulate object formed of the highly crystalline rare earth metal fluoride particulate object having an average primary particle diameter measured by the electron microscope observation of from 1 nm to 500 nm in the dispersion medium, to the mechanochemical treatment to provide the treated slurry liquid in which the low crystalline rare earth metal fluoride particulate object is dispersed in the dispersion medium; and subjecting the treated slurry thus obtained to spray drying. Here, with regard to the primary raw material particulate object and the secondary raw material particulate object, it is acceptable to include a trace amount of a substance except crystalline rare earth metal fluoride particles, such as a surface treatment agent or additive that has physically adhered, or been chemically bonded, to the surface of each of the crystalline rare earth metal fluoride particles.

(1-1) Primary Raw Material Particulate Object

A rare earth metal fluoride, which has been generally used as an X-ray opaque material, or commercial rare earth metal fluoride powder having a full width at half maximum of less than 0.3° (specifically, from about 0.17° to about 0.27°), which is commercially available, may be used as the primary raw material particulate object (highly crystalline rare earth metal fluoride particulate object) without any particular limitation. The full width at half maximum of the primary raw material particulate object is preferably measured by performing X-ray diffraction measurement as required.

In addition, particles whose surfaces have been subjected to coating treatment with nano silica or the like, or particles subjected to surface treatment with a silane coupling agent or the like may be used as the crystalline rare earth metal fluoride particles to be used as the primary raw material particulate object.

In the mechanochemical treatment in the secondary raw material particulate object-preparing step, the following may occur depending on treatment conditions: when a treatment time is lengthened, the pulverization of the particles in the primary raw material particulate object occurs to disintegrate secondary particles (aggregated particles) and primary particles, and hence the diameters of these particles reduce. However, when the treatment time is about several hours, no large changes in particle diameters of the primary particles and aggregated particles at a sub-micron level are observed, though the disintegration of coarse aggregated particles occurs. In view of the foregoing, a particulate object having an average primary particle diameter measured by electron microscope observation of from 1 nm to 500 nm is used as the primary raw material particulate object, and a particulate object having an average primary particle diameter of from 5 nm to 300 nm is preferably used. In addition, a particulate object having an average particle diameter measured by a laser diffraction/scattering method, which is capable of measuring even the diameter of an aggregated particle, of from 0.1 $\mu$m to 1 $\mu$m, in particular, from 0.1 $\mu$m to 0.6 $\mu$m, is preferably used, and a particulate object having an average particle diameter of from 0.1 $\mu$m to 0.3 $\mu$m is more preferably used. The average primary particle diameter is a value measured with a scanning electron microscope. Specifically, the powder was observed with an electron microscope at a magnification of 100,000, and the average of the diameters of 100 primary particles in the resultant observation image was defined as the average primary particle diameter.

(1-2) Mechanochemical Treatment

The mechanochemical treatment means treatment including applying mechanical energy to the primary raw material particulate object, and specifically means at least one kind of treatment selected from the group consisting of: mechanical frictional crushing; pulverization; and dispersion. From the viewpoint that the full width at half maximum, which serves as the degree of crystal perfection of the mechanochemically treated rare earth metal fluoride particles, is reliably and efficiently controlled to a desired value with ease, a wet method is preferably adopted as a method for the mechanochemical treatment, and a treatment method including using a wet bead mill is particularly preferred. When the mechanochemical treatment is performed by the wet method, although a solvent, such as water or an alcohol, a polymerizable monomer, or the like may be used as a medium, a medium that is liquid at normal temperature (from 15°C to 25°C) is preferred from the viewpoint of, for example, the dispersibility of the raw material powder.

The mechanochemical treatment using the wet bead mill is described in detail below.

In the mechanochemical treatment using the wet bead mill, a slurry obtained by mixing the raw material powder to be subjected to the mechanochemical treatment and a medium is brought into contact with media (beads) to which movement has been applied through stirring, vibration, or the like. Thus, the raw material powder is pulverized and disintegrated. A material for the beads to be used as the media is, for example, glass, alumina, zircon, zirconia, steel, or a resin, but is preferably alumina or zirconia because of its excellent abrasion resistance and relatively low contamination. The sizes of the beads to be used only need to be selected in accordance with the target particle diameter of the X-ray opaque filler without any particular limitation. In general, however, beads each having a diameter of from 0.01 mm to 0.5 mm are preferably used. The beads each having such diameter are also suitable for obtaining an X-ray opaque filler having a particle diameter preferred for addition to a dental curable composition.

Examples of the wet bead mill include, according to its operation mode: a batch mode in which treatment is performed by directly loading the slurry and the beads into a device; a circulation mode in which the slurry is circulated between a tank and a device; and a pass mode in which the slurry is passed through a device at predetermined times. Those operation modes only need to be selected in accordance with the amount of the raw material powder to be used in the mechanochemical treatment. A bead mill of a circulation mode is preferably used because the bead mill has satisfactory productivity and can treat the raw material powder in a relatively large amount.

In some of the operation modes, such as the circulation mode and the pass mode described above, the slurry and the beads are required to be separated from each other at the time of the performance of the mechanochemical treatment. A bead separation mode is, for example, a slit mode, a screen mode, or a centrifugal separation mode. Those bead separation modes only need to be selected in accordance with the particle diameters of the beads to be used, and any of the modes may be used without any particular limitation. The concentration of the slurry to be used in the mechanochemical treatment is preferably as follows: the amount of the raw material powder is 50 parts by mass or less with respect to 100 parts by mass of the medium. When the amount of the raw material powder in the slurry is more than 50 parts by mass, the viscosity of the slurry increases, and hence the mechanochemical treatment may become difficult.

The increase in viscosity of the slurry can be suppressed by adding a dispersant to the slurry. Accordingly, when the dispersant is added to the slurry, the slurry having a higher concentration can be subjected to the mechanochemical treatment. Any known surfactant may be used as the dispersant to be used without any particular limitation, and examples thereof include a nonionic surfactant, an anionic surfactant, a cationic surfactant, an ampholytic surfactant, and a polymer-based surfactant thereof. Specific examples thereof include a glycerin fatty acid ester and an alkylene glycol adduct thereof, an aliphatic monocarboxylic acid salt, an alkyl amine salt, and an alkyl betaine. When granulation is performed by mixing the raw material powder subjected to the mechanochemical treatment with a raw material for forming the resin matrix (resin matrix raw material), a cationic surfactant is preferably used from the viewpoint of the dispersibility of the powder at the time of the mixing.

Conditions for the mechanochemical treatment vary depending on conditions, such as the operation mode of the wet bead mill apparatus to be used, the diameter of each of the beads, the full width at half maximum of the raw material powder, and the concentration of the slurry. Those conditions only need to be appropriately selected after a preliminary experiment has been performed with an apparatus, with which the mechanochemical treatment is to be actually performed, to identify the full width at half maximum of the raw material powder after the mechanochemical treatment with respect to a mechanochemical treatment time. In addition, when the mechanochemical treatment is performed, mechanochemically treated secondary raw material powder having a desired full width at half maximum may be obtained by: appropriately sampling a slurry during the mechanochemical treatment as required; and appropriately identifying the full width at half maximum of the slurry.

Although the full width at half maximum of the secondary raw material particulate object only needs to be 0.3° or more, the full width at half maximum is preferably 0.35° or more, more preferably 0.4° or more from the viewpoint that the X-ray opaque filler of this embodiment is obtained more stably and more reliably. The upper limit value of the full width at half maximum of the secondary raw material particulate object is not particularly limited, and may be appropriately selected in accordance with the target value of the full width at half maximum of the X-ray opaque filler to be produced. In practical use, however, the upper limit value is preferably 40° or less, more preferably 1° or less.

(1-3) Spray Drying and Secondary Raw Material Particulate Object

The slurry obtained by the mechanochemical treatment, the slurry having dispersed therein the secondary raw material particulate object (mechanochemically treated rare earth metal fluoride particulate object) whose full width at half maximum has been adjusted to 0.3° or more, is subjected to spray drying. Thus, the secondary raw material particulate object formed of the low crystalline rare earth metal fluoride particulate object is recovered.

Herein, the spray drying means a step of spraying a liquid raw material (slurry) into hot air to instantly evaporate its moisture, to thereby provide dry powder (granule), and means, for example, that the secondary raw material particulate object is obtained by subjecting the mechanochemically treated slurry to spray drying with an atomizer disc-type spray dryer.

The secondary raw material particulate object that has undergone the mechanochemical treatment step and the posttreatment step may be subjected to surface treatment for improving its affinity for the resin matrix raw material to be used in the granulation of the organic-inorganic composite particles. A compound to be generally used, such as a silane coupling agent or a titanate coupling agent, may be used as a surface treatment agent to be used in the surface treatment.

(2) Curable Raw Material Composition-preparing Step

In the curable raw material composition-preparing step, the curable raw material composition is obtained by kneading 100 parts by mass of the polymerizable monomer serving as a raw material for the resin matrix and 150 parts by mass to 900 parts by mass of the secondary raw material particulate object under a pressure of from 1.0 to $1.0 \times 10^5$ (Pa). When the loading ratio of the low crystalline rare earth metal fluoride particulate object to be loaded into the specific X-ray opaque filler is high, at the time of the addition of the specific X-ray opaque filler produced by the production method of the present invention to a dental curable composition for the purpose of imparting X-ray radiography contrast to a cured body of the composition, the X-ray radiography contrast can be more efficiently improved. Accordingly, the amount of the secondary raw material particulate object to be added to 100 parts by mass of the polymerizable monomer is preferably as large as possible, and the effect of the present invention becomes more remarkable as the amount increases. Meanwhile, as the amount of the secondary raw material particulate object to be added to the polymerizable monomer increases, a kneading time required for the curable raw material composition-preparing step becomes longer. Accordingly, the amount of the secondary raw material particulate object to be added in the production method of the present invention is set to preferably from 233 parts by mass to 900 parts by mass, particularly preferably from 250 parts by mass to 567 parts by mass with respect to 100 parts by mass of the polymerizable monomer.

(2-1) Polymerizable Monomer serving as Raw Material for Resin Matrix

Although a known polymerizable monomer such as a radical polymerizable monomer may be used as the polymerizable monomer serving as a raw material for the resin matrix of each of the organic-inorganic composite particles, a radical polymerizable monomer such as a (meth)acrylate-based monomer to be used in a dental curable composition is preferably used.

The refractive index $n_X$ of the crystalline rare earth metal fluoride particles to be used as the primary and secondary raw material particulate objects for a sodium d-line at 25°C typically falls within the range of from 1.50 to 1.65. In the case of crystalline ytterbium fluoride particles, the refractive index is 1.55. Accordingly, the refractive index $n_{(F-MX)}$ of the resin material, which is a main component for the resin matrix for forming each of the organic-inorganic composite particles (particle body), for the sodium d-line at 25°C is preferably from 1.45 to 1.60. The term "resin material, which is a main component for the resin matrix" as used herein means a cured product of the polymerizable monomer (a cured product of a composition formed only of the polymerizable monomer or a cured product of a composition formed of the polymerizable monomer and a small amount of a polymerization initiator). When the $n_X$ and the $n_{(F-MX)}$ have values approximate to each other as described above, an improvement in transparency of the curable composition using the X-ray opaque filler is further facilitated.

From such viewpoint, the following formula (1A) is preferably satisfied, the following formula (2A) is more preferably satisfied, and the following formula (3A) is still more preferably satisfied.

$$\cdot \text{Formula (1A)} \qquad -0.02 \leq (n_X - n_{(F-MX)}) \leq 0.1$$
$$\cdot \text{Formula (2A)} \qquad 0.01 \leq (n_X - n_{(F-MX)}) \leq 0.07$$
$$\cdot \text{Formula (3A)} \qquad 0 \leq (n_X - n_{(F-MX)}) \leq 0.05$$

Further, when the specific X-ray opaque filler is blended into an existing structural color-based dental curable composition, it is required to use such a resin material that the absolute value $|n_{(MX)} - n_{(F-MX)}|$ of the difference between the refractive index $n_{(MX)}$ of the cured body of the polymerizable monomer in the existing structural color-based dental curable composition for the sodium d-line at 25°C and the refractive index $n_{(F-MX)}$ of the resin material for forming the resin matrix of the particle body for the sodium d-line at 25°C is from 0 to 0.1 in order that the structural color-expressing effect of the composition may not impaired. Accordingly, a polymerizable monomer having composition identical or similar to that of the polymerizable monomer in the existing structural color-based dental curable composition is preferably used.

In addition, a catalytic amount of a polymerization initiator is typically blended into a raw material composition for granulating the curable raw material composition. A thermal polymerization initiator is suitable as the polymerization initiator, and a chemical polymerization initiator and/or a photopolymerization initiator may be used in combination therewith. Further, any other additive may be used as required. The same materials as those used in the present dental curable composition production method to be described later may be utilized as specific examples of the polymerizable monomer, the polymerization initiator, and the other additive.

(2-1) Kneading Conditions

In the curable raw material composition-preparing step, in order to efficiently obtain the curable raw material composition, it is required to knead predetermined amounts each of the polymerizable monomer and the secondary raw material particulate object under a pressure of from 1.0 to $1.0 \times 10^5$ (Pa) (under reduced pressure), which is lower (reduced pressure state) than 101,325 (Pa) considered to be atmospheric pressure, in terms of absolute pressure. Herein, the absolute pressure means a pressure defined with respect to a complete vacuum (absolute vacuum) unlike a so-called gauge pressure. The kneading under a pressure of from 1.0 to $1.0 \times 10^5$ (Pa) in terms of absolute pressure means that an atmospheric pressure at the time of the actual kneading is controlled within the above-mentioned pressure range. When a kneading atmosphere pressure is less than 1.0 (Pa), the pressure becomes so low that the polymerizable monomer and the secondary raw material particulate object scatter to establish a state in which the curable raw material composition cannot be kneaded. When the kneading atmosphere pressure is more than $1.0 \times 10^5 = 100,000$ (Pa)=100 (kPa), the pressure is so high that the polymerizable monomer does not infiltrate the secondary raw material particulate object, and hence a state in which the curable raw material composition cannot be kneaded is established. From the viewpoints of the effect and the short time period required to adjust the atmosphere to a predetermined pressure, the kneading atmosphere pressure is set to preferably from 100 to 35,000 (Pa), particularly preferably from 1,000 to 33,000 (Pa).

Such control of the atmospheric pressure at the time of the kneading can be performed by using a kneading machine such as a planetary mixer, which can perform kneading in a sealable space having pressure resistance, as follows: after the respective raw materials (to be) kneaded have been loaded, a pressure in the space is set to a predetermined pressure with a decompression pump or the like, and then the space is sealed, followed by the start of the kneading. For example, the foregoing may be suitably performed with a twin-screw planetary type kneading apparatus such as a planetary mixer, which enables kneading under a reduced pressure state, by adopting a vacuum hood and a vacuum shaft seal.

Although a temperature at the time of the kneading is not particularly limited, the temperature is set to preferably from 25°C to 60°C, more preferably from 30°C to 40°C.

(3) Curing Step

In the curing step, the curable raw material composition is cured to provide the bulk material formed of the composite material obtained by dispersing the low crystalline rare earth metal fluoride particles in the resin matrix. The curable raw material composition obtained through the kneading step is preferably cured by heat treatment. A heating temperature is preferably from 50°C to 500°C, more preferably from 80°C to 200°C. In addition, to improve the polymerization ratio of a cured product to be obtained and to reduce polymerization inhibition by oxygen, a heating reaction is preferably performed in an inert gas such as a nitrogen gas together with the heat treatment. A heat treatment time is preferably from 10 minutes to 500 minutes, more preferably from 20 minutes to 200 minutes.

(4) Granulation Step

In the granulation step, the bulk material is pulverized and then the particle size adjustment is performed so that the average particle diameter becomes from 3 $\mu$m to 110 $\mu$m. A method of pulverizing the bulk material is not particularly limited, and for example, a ball mill may be used. When a ball mill is used as a method for the pulverization, a material for balls to be used in the pulverization is preferably zirconia for the purpose of reducing contamination. The particle diameter of each of zirconia balls to be used is preferably from 1 mm to 100 mm, preferably from 10 mm to 50 mm. A pulverizing time in the ball mill is preferably from 5 minutes to 120 minutes, more preferably from 30 minutes to 90 minutes.

The particle size adjustment to be performed after the performance of the pulverization step may be suitably performed with a sieve having an aperture corresponding to the target particle size (average particle diameter). In addition, the organic-inorganic composite particles (particle body) obtained after the pulverization may be subjected to various kinds of surface treatment, coating treatment, external additive treatment, or the like as required.

3. The Present Dental Curable Composition Production Method

(1) Overview of the Present Dental Curable Composition Production Method

The present dental curable composition production method is mainly characterized in that a predetermined amount of the specific X-ray opaque filler produced by the present X-ray opaque filler production method, the filler including organic-inorganic composite particles (particle body) each using, as a resin material for forming its resin matrix, such a resin material that the $|n_{(MX)}-n_{(F-MX)}|$ is from 0 to 0.1 is blended into the dental curable composition (existing structural color-based dental curable composition) used as a conventionally known structural color-based CR.

(1-1) Existing Structural Color-based Dental Curable Composition

As described above as the related art, the term "existing structural color-based dental curable composition" as used herein means a dental curable composition including a predetermined amount of each of a polymerizable monomer, an inorganic filler, and a polymerization initiator, the dental curable composition being allowed to express a structural color, which develops a predetermined color tone independent of the incidence angle of light, through the interference, scattering, or the like of the light as follows: a filler containing spherical inorganic particles having a specific average particle diameter and a specific particle size distribution, that is, one or a plurality of "groups of spherical particles having identical diameters" (G-PIDs) are used, and the refractive index of the spherical inorganic particles is made larger than the refractive index of a resin portion serving as a matrix at the time of the curing of the composition; or specifically, the following conditions 1 to 3 are satisfied when the amount of the polymerizable monomer in the dental curable composition is set to 100 parts by mass.

Condition 1: the composition includes: 10 parts by mass to 1,500 parts by mass in total of one or a plurality of "groups of spherical particles having identical diameters" (G-PIDs), which are each formed of an aggregate of inorganic spherical particles having a predetermined average primary particle diameter in a range of from 100 nm to 1,000 nm, and in each of which the individual inorganic spherical particles for forming the aggregate include substantially the same substance, and 90% or more of the total number of the particles are present in a range of $\pm 5\%$ of the predetermined average primary particle diameter in a number-based particle size distribution of the aggregate; and 0.01 part by mass to 0.5 part by mass of a polymerization initiator.

Condition 2: when the number of the one or the plurality of "groups of spherical particles having identical diameters" is represented by "a", and the respective "groups of spherical particles having identical diameters" are each represented by $G\text{-}PID_m$ in order of increasing average primary particle diameter, provided that when "a" represents 1, "m" represents 1, and when "a" represents 2 or more, "m" represents a natural number of from 1 to "a", the substances for forming the individual particles of the respective $G\text{-}PID_m$s in a case where the "a" represents 2 or more may be different from each other, and the average primary particle diameters of the respective $G\text{-}PID_m$s in the case are different from each other by 25 nm or more.

Condition 3: when a refractive index of a cured body of the polymerizable monomer for a sodium d-line at 25°C is represented by $n_{(MX)}$, and refractive indices of the inorganic spherical particles for forming the respective $G\text{-}PID_m$s for the sodium d-line at 25°C are each represented by $n_{(G\text{-}PIDm)}$, the following relationship is valid for any one of the $n_{(G\text{-}PIDm)}$s.

$$n_{(MX)} < n_{(G\text{-}PIDm)}$$

In addition, in the present dental curable composition production method, there is produced a dental curable composition, which can provide a cured body that expresses a structural color having a predetermined color tone independent of the incident angle of light, by mixing the following components so that the conditions 1 to 3 may be satisfied: 100 parts by mass of the polymerizable monomer; 10 parts by mass to 1,500 parts by mass in total of the one or the plurality of "groups of spherical particles having identical diameters" (G-PIDs); 1 part by mass to 100 parts by mass of the specific X-ray opaque filler produced by the present X-ray opaque filler production method, the filler including the organic-inorganic composite particles (particle body) each using such resin material that the absolute value $|n_{(MX)}\text{-}n_{(F\text{-}MX)}|$ is from 0 to 0.1, in terms of the total mass of the low crystalline rare earth metal fluoride particles; and 0.01 part by mass to 0.5 part by mass of the polymerization initiator.

(1-2) Features of Dental Curable Composition obtained by the Present Dental Curable Composition Production Method

The dental curable composition produced by the present dental curable composition production method can impart high X-ray opacity to a cured body of the dental curable composition without impairment of the following excellent features of the existing structural color-based dental curable composition: "when the composition is used as a CR, (i) a problem of discoloration over time after treatment hardly occurs because the composition does not use any dye substance or pigment substance; (ii) the composition expresses a structural color having a specific color tone, which is independent of the incidence angle of light, in accordance with the average particle diameter of the spherical inorganic particles to be used, and particularly when spherical inorganic particles having an average primary particle diameter of from 230 nm to 350 nm are used, the cured body can be colored to from a yellow color to a red color serving as a color similar to a dentin color; and (iii) the cured body has moderate transparency and hence easily harmonizes with the color of a restoration target tooth, and thus the restoration target teeth having a wide variety of colors can each be restored so as to have an appearance close to that of a natural tooth with one kind of composite resin without performance of complicated shade taking or the selection of the shade of the composite resin."

Such features of the dental curable composition result from the fact that a state in which the structural color is expressed in the cured body thereof is substantially the same as that in a cured body of the existing structural color-based dental curable composition free of the specific X-ray opaque filler and a filler that adversely affects the expression of the structural color (also referred to as "base existing structural color-based dental curable composition"). In addition, such properties may be recognized by comparing the spectral reflectance ratios "$SR_1/SR_2$" of the cured body of the dental curable composition obtained by the present dental curable composition production method and the cured body of the base existing structural color-based dental curable composition to each other. Herein, the above-mentioned $SR_1$ and $SR_2$ mean the following maximum values: the maximum value ($SR_1$) of a spectral reflectance in the wavelength region of from 600 nm or more to 750 nm or less (yellow to red region) in the spectral reflectance curve of a 1-millimeter thick cured body, which is obtained by curing each of the dental curable compositions, under a black background, the curve being measured with a color-difference meter; and the maximum value ($SR_2$) of the spectral reflectance in the wavelength region of from 400 nm or more to 500 nm or less (blue region) therein. A smaller spectral reflectance ratio means that the structural color (colored light) of the cured body becomes more bluish, and a larger spectral reflectance ratio means that the structural color (colored light) of the cured body becomes more reddish.

In the base existing structural color-based dental curable composition, the cured body thereof is typically caused to express an expected structural color (provide an expected spectral reflectance ratio, i.e., ratio "$SR_1/SR_2$") by controlling the average primary particle diameter of each of the G-PIDs to be blended thereinto. For example, in order that the composition may have excellent color tone compatibility when used in the restoration of a cavity formed in dentin or over the range of from enamel to the dentin, the spectral reflectance ratio is typically set within the range of from 0.9 to 1.5. This is because of the following reasons: when the spectral reflectance ratio is less than 0.8, a yellowish to reddish structural color (colored light) is weak, and hence it tends to be difficult to obtain color tone compatibility with a natural tooth having a yellow to red tint, that is, a restored tooth including the dentin; and when the spectral reflectance ratio is more than 2.0, it becomes difficult to obtain satisfactory color tone compatibility because the yellow to red tint of the cured body is excessively strong as compared to that of the natural tooth.

The spectral reflectance ratio of the above-mentioned dental curable composition produced by the present dental curable composition production method is hardly changed despite the fact that the cured body thereof is caused to show high X-ray opacity by blending the specific X-ray opaque filler into the base existing structural color-based dental curable composition. Accordingly, even when a toning agent such as a pigment is not particularly blended into the composition, the spectral reflection ratio can be maintained at a value of, for example, 0.9 or more.

In addition, the above-mentioned feature (iii) is further described. According to the above-mentioned dental curable composition produced by the present dental curable composition production method, there can be obtained, for example, a cured body having a contrast ratio C of from 0.20 to 0.50, preferably a cured body having a contrast ratio C of from 0.25 to 0.45. Herein, the contrast ratio C is defined as the ratio "$Y_b/Y_w$" of the $Y_b$ of a cured body sample having a thickness of 1 mm, which is a Y-value measured with a color-difference meter under a black background, to the $Y_w$ thereof, which is a Y-value measured therewith under a white background, the ratio serving as an indicator of the transparency of the cured body of the dental curable composition. A smaller contrast ratio means higher transparency.

When the contrast ratio of the cured body of the above-mentioned dental curable composition is less than 0.20, the following is conceivable: the brightness (color shades) of the cured body in a filled site reduces, and hence transmitted light in the filled site strengthens and the colored light from the cured body weakens; accordingly, in the case where a deep cavity (e.g., a class IV cavity) is filled with the dental curable compositon, color tone compatibility serving as an effect of the present invention is hardly obtained. Meanwhile, when the contrast ratio of the cured body is more than 0.50, the following is conceivable: the brightness of the cured body increases to make it difficult for light to reach a restoration serving as a ground, and hence reflected light on the surface of the filled site strengthens and the colored light from the cured body weakens; accordingly, the color tone compatibility serving as the effect of the present invention is hardly obtained. That is, the contrast ratio C of the cured body of the curable composition preferably falls within the range of from 0.20 to 0.50, and more preferably falls within the range of from 0.20 to 0.45 in order that the cured body may have excellent color tone compatibility irrespective of the depth of a cavity to be restored.

The reason why such effect is obtained is not necessarily clear, and the present invention is by no means limited by logic. However, the inventors of the present invention have assumed the reason as described below. That is, with regard to the fact that a small blending amount of the low crystalline rare earth metal fluoride particulate object provides high X-ray radiography contrast, the following is conceivable: when the low crystalline rare earth metal fluoride particulate object is blended as it is into the dental curable composition, the individual particles each having weak (small) X-ray opacity are dispersed in a uniform manner, and hence X-ray opacity as a whole blurs; in contrast, when the particulate object is blended as the organic-inorganic composite particles, the particles are uniformly dispersed (spattered) over the entirety of a "region in which the density of the rare earth metal fluoride particles is locally high" (region locally having high X-ray opacity), and hence the particles can be clearly identified when viewed as an image. In addition, with regard to the fact that the specific X-ray opaque filler do not adversely affect the expression of the expected structural color when blended into the structural color-based CR, the following is conceivable: in the case where the low crystalline rare earth metal fluoride particulate object is blended as it is, the particles enter a space between the particles of the group of spherical particles having an identical diameter (G-PID), the group forming an ideal periodic structure, to disturb the periodic structure; meanwhile, in the case where the low crystalline rare earth metal fluoride particulate object is turned into the organic-inorganic composite particles, the absolute number of the particles reduces as compared to that in the case where the low crystalline rare earth metal fluoride particulate object is blended as it is, to thereby reduce the frequency at which the periodic structure of the G-PID is disturbed, and the organic-inorganic composition increases the diameters of the particles to make it difficult for the particles to enter the space in the periodic structure of the G-PID. That is, it is conceivable that when the low crystalline rare earth metal fluoride particulate object is turned into the organic-inorganic composite particles, the periodic structure formed by the G-PID is hardly disturbed, and hence the expression of the expected structural color is not adversely affected.

(2) Raw Materials to be used in the Present Dental Curable Composition Production Method

The raw materials to be used in the present dental curable composition production method, their blending amounts, and the like are basically the same as the components in the existing structural color-based dental curable composition disclosed in each of Patent Literatures 1 and 2, their blending amounts, and the like except that the predetermined amount of the specific X-ray opaque filler is blended. In view of the foregoing, here, the dental curable composition of the present invention is described after those components have been simply described.

(2-1) Polymerizable Monomer

Although a polymerizable monomer that may be used in a related-art dental curable composition may be used as the polymerizable monomer without any particular limitation, a (meth)acrylate-based monomer is preferably used. Specific examples of the (meth)acrylate-based monomer that may be suitably used may include methyl (meth)acrylate, glycidyl (meth)acrylate, 2-cyanomethyl (meth)acrylate, polyethylene glycol mono(meth)acrylate, allyl (meth)acrylate, 2-hydroxyethyl mono(meth)acrylate, ethylene glycol di(meth)acrylate, diethylene glycol di(meth)acrylate, triethylene glycol di(meth)acrylate (3G), nonaethylene glycol di(meth)acrylate, propylene glycol di(meth)acrylate, dipropylene glycol di(meth)acrylate, 2,2-bis[4-(meth)acryloyloxyethoxyphenyl]propane, 2,2-bis[4-(meth)acryloyloxyethoxyethoxyphenyl] propane, 2,2-bis{4-[3-(meth)acryloyloxy-2-hydroxypropoxy]phenyl}propane, 1,4-butanediol di(meth)acrylate, 1,3-hexanediol di(meth)acrylate, 1,6-bis(methacrylethyloxycarbonylamino)-2,2,4-trimethylhexane (UDMA), and trimethylolpropane di(meth)acrylate. Two or more kinds of those polymerizable monomers may be used in combination as appropriate.
Of those polymerizable monomers, a bifunctional to tetrafunctional polymerizable monomer is preferred because of, for example, the following reasons: the monomer has high polymerizability and particularly improves the mechanical strength of the cured body. From the viewpoint of the transparency of the cured body of the curable composition, it is more preferred that any one of an ethylene glycol-based di(meth)acrylate, 2,2-bis[4-(meth)acryloyloxyethoxyphenyl]propane, 2,2-bis{4-[3-(meth)acryloyloxy-2-hydroxypropoxy]phenyl}propane, and 1,6-bis(methacrylethyloxycarbonylamino)-2,2,4-trimethylhexane (UDMA) be used, or two or more kinds of those polymerizable monomers be used in combination.
From the viewpoint that the condition 3 is easily satisfied, that is, from the viewpoint that the condition related to the refractive indices is easily satisfied, the kinds and amounts of the polymerizable monomers are desirably set so that the refractive index of a polymerizable monomer composition (mixture) for a sodium d-line at 25°C may fall within the range of from 1.38 to 1.55. That is, when a silica-titanium group element oxide-based composite oxide whose refractive index is easily adjusted is used as the inorganic spherical particles, the refractive index thereof for the sodium d-line at 25°C falls within the range of from about 1.45 to about 1.58 depending on the content of the silica content thereof. However, when the refractive index of the polymerizable monomer composition is set within the range of from 1.38 to 1.55, the refractive index of the cured body to be obtained can be adjusted within the range of from about 1.40 to about 1.57, and hence it becomes easier to satisfy the condition. The refractive index of the polymerizable monomer or the cured body of the polymerizable monomer may be determined by using an Abbe refractometer at 25°C.
In addition, from the viewpoint of preventing a reduction in transparency of the cured body of the dental curable composition, the $|n_{(MX)}-n_{(F-MX)}|$ needs to be set to from 0 to 0.1, and is set to preferably from 0 to 0.05. To adjust the refractive indices as described above, for example, the same kind of polymerizable monomer is preferably used as the polymerizable monomer to be used in the curable composition and a polymerizable monomer to be used in the production of the resin material for forming the resin matrix of each of the organic-inorganic composite particles. In addition, when the two or more kinds of polymerizable monomers are used in combination, a blending ratio therebetween in the composition is preferably set to a value equal or approximate to that in the resin material.

(2-2) Group of Spherical Particles having Identical Diameter: G-PID

The meaning of the group of spherical particles having an identical diameter "G-PID" is as follows: the G-PID is formed of an aggregate of inorganic spherical particles having a predetermined average primary particle diameter in the range of from 100 nm or more to 1,000 nm or less (from 100 nm to 1,000 nm); the individual inorganic spherical particles for forming the aggregate include substantially the same substance; and 90% or more of the total number of the particles are present in the range of ±5% of the predetermined average primary particle diameter in the number-based particle size distribution of the aggregate.
The term "average primary particle diameter of the inorganic spherical particles" as used herein means an average determined as follows: a photograph of the G-PID is taken with a scanning electron microscope; 100 or more particles observed in the unit field of view of the photograph are selected; and the primary particle diameters (maximum diameters) of the respective particles are determined, followed by their averaging. In addition, the term "spherical" only needs to mean that the particles are substantially spherical, and the particles are not necessarily required to be complete true spheres. An average evenness determined as follows only needs to be preferably 0.6 or more, more preferably 0.8 or more: a photograph of the G-PID is taken with the scanning electron microscope; the maximum diameters of the respective particles (100 or more particles) present in the unit field of view of the photograph are measured; and an evenness is determined by dividing a particle diameter in a direction perpendicular to each of the maximum diameters by the maximum diameter, followed by the averaging of the determined values. The G-PID has such a particle size distribution as described above, and hence the average primary particle diameter of the G-PID, for which a primary particle suspension is obtained by ultrasonic irradiation, coincides well with a particle diameter (D50p value) corresponding to a cumulative 50% from smaller diameters, the particle diameter being determined from the number particle size distribution of the above-mentioned suspension measured with a particle size distribution meter.

In each of the cured bodies of the existing structural color-based dental curable composition and the dental curable composition obtained by the present dental curable composition production method, the respective constituent particles of the G-PID are dispersed in the resin matrix while having a specific short-distance ordered structure. Thus, diffraction interference occurs in accordance with a Bragg condition, and hence light having a specific wavelength is emphasized to generate colored light having a color tone in accordance with the average primary particle diameter (to express a structural color). That is, the expression of the structural color requires that 90% (number) or more of the inorganic spherical particles for forming the G-PID be present in the range of $\pm 5\%$ of the average primary particle diameter. That is, when the ratio (%) of "the number of the particles present in the range of $\pm 5\%$ of the average primary particle diameter" to "the total number of the particles for forming the G-PID" is defined as a "5% particle percentage content," the 5% particle percentage content needs to be 90% or more. In addition, to express a structural color having a specific color tone in a wide range of from a bluish color to a reddish color including a yellowish color, the average primary particle diameter of the inorganic spherical particles for forming the G-PID needs to fall within the range of from 100 nm to 1,000 nm. When spherical particles having an average primary particle diameter of less than 100 nm are used, the interference phenomenon of visible light hardly occurs, and the expression of the structural color also hardly occurs. Meanwhile, a case in which spherical particles having an average primary particle diameter of more than 1,000 nm are used is not preferred because the sedimentation of the spherical particles or a reduction in abradability of the cured body occurs, though the expression of the light interference phenomenon can be expected.

When the average primary particle diameter is from 230 nm to 800 nm, a yellowish to reddish structural color (colored light) is easily expressed, and when the average primary particle diameter is from 150 nm to less than 230 nm, a bluish structural color (colored light) is easily expressed. The average primary particle diameter of the G-PID is suitably from 230 nm to 800 nm, more suitably from 240 nm to 500 nm, particularly suitably from 260 nm to 350 nm because the yellowish to reddish structural color (colored light) preferred as a dental filling restoration material is expressed. When a G-PID having an average primary particle diameter in the range of from 230 nm to 260 nm is used, colored light to be obtained is yellowish, and is useful in the restoration of a tooth falling into the category of a B type (reddish yellow color) in a shade guide ("VITA Classical", manufactured by VITA Zahnfabrik H. Rauter GmbH & Co. KG), particularly in the restoration of a cavity formed over the range of from enamel to dentin. In addition, when a G-PID having an average primary particle diameter in the range of from 260 nm to 350 nm is used, colored light to be obtained is reddish, and is useful in the restoration of a tooth falling into the category of an A type (reddish brown color) in the shade guide ("VITA Classical", manufactured by VITA Zahnfabrik H. Rauter GmbH & Co. KG), particularly in the restoration of a cavity formed over the range of from the enamel to the dentin. The hue of the dentin is often such a reddish color. Accordingly, an aspect in which only the G-PID having an average primary particle diameter in the range of from 260 nm to 350 nm is used is most preferred because satisfactory compatibility with a wide variety of restored teeth having various color tones is obtained. Meanwhile, when only a G-PID having an average primary particle diameter in the range of from 150 nm to less than 230 nm is used, as described above, the colored light to be obtained is bluish, and is liable to be unsatisfactory in color tone compatibility with a tooth structure in a cavity formed over the range of from the enamel to the dentin, but is useful in the restoration of the enamel, particularly in the restoration of an incisal portion.

The number of the kinds of the G-PIDs to be used may be one or two or more. The number "a" of the G-PIDs to be incorporated is preferably from 1 to 5, particularly preferably from 1 to 3, most preferably 1 or 2. However, when the inorganic particles include a plurality of kinds of G-PIDs, the average primary particle diameters of the respective G-PIDs need to be different from each other by 25 nm or more. That is, when the number of the G-PIDs in the inorganic particles is represented by "a" (e.g., "3"), and the respective G-PIDs are each represented by G-PID$_m$ in order of increasing average primary particle diameter (provided that when "a" represents 1, "m" represents 1, and when "a" represents 2 or more, "m" represents a natural number of from 1 to "a"), substances for forming the individual particles in the respective G-PID$_m$s (e.g., a G-PID$_1$, a G-PID$_2$, and a G-PID$_3$ when a=3) may be different from each other. However, when the average primary particle diameters of the respective G-PID$_m$s in the case are each represented by $d_m$, the respective $d_m$s need to be different from each other by 25 nm or more (e.g., when a=3, $|d_1-d_2|\geq 25$ nm, $|d_2-d_3|\geq 25$ nm, and of course, $|d_1-d_3|\geq 25$ nm). As a result of the satisfaction of the condition, a structural color peculiar to each G-PID (in accordance with its average primary particle diameter) can be expressed probably because of, for example, the following reason: each G-PID is, for example, dispersed in a form like an aggregate in which the inorganic spherical particles whose number is as small as not more than about 20 are aggregated by an extremely loose bonding force, and hence the inorganic spherical particles can be dispersed while having a short-distance ordered structure, which can express a structural color for each G-PID. In contrast, when the condition is not satisfied, it becomes difficult to express the structural color probably because of the following reason: the particle diameter distribution of the entirety of the inorganic spherical particles broadens; perhaps as a result of the foregoing, the inorganic spherical particles for forming each G-PID are dispersed while replacing each other, and hence the same phenomenon as that at the time of the use of a single aggregate of inorganic spherical particles that does not satisfy the condition of a number-based particle size distribution occurs. When the plurality of G-PIDs are used, the average primary particle diameters $d_m$ of the respective G-PID$_m$s are different from each other by preferably 30 nm or more, particularly preferably 40 nm or more.

When a plurality of kinds of G-PIDs are used, the respective G-PIDs have extremely sharp particle size distributions, and such a difference as described above is present between their average primary particle diameters. Accordingly, the particle size distributions of the respective G-PIDs hardly overlap each other, and even when the particle size distributions of the respective G-PIDs partly overlap each other, the distributions can be identified.

The G-PID is preferably such that its inorganic spherical particles aggregate to form an aggregated particle because the dispersed state of the inorganic spherical particles easily obtains the above-mentioned short-distance ordered structure. For example, the average aggregated particle diameter of the G-PID preferably falls within the range of from 5 $\mu$m to 200 $\mu$m, and more preferably falls within the range of from 10 $\mu$m to 100 $\mu$m. The term "average aggregated particle diameter of the G-PID" means a volume median diameter determined on the basis of the results of measurement by a laser diffraction/scattering method with a particle size distribution meter.

(2-3) Inorganic Spherical Particles for forming G-PID

A material for the inorganic spherical particles for forming the G-PID is not particularly limited as long as the conditions for forming the G-PID are satisfied. Examples of the material that may be suitably used may include materials formed of amorphous silica, silica-titanium group element oxide-based composite oxide particles (e.g., silica-zirconia and silica-titania particles), quartz, alumina, barium glass, strontium glass, lanthanum glass, fluoroaluminosilicate glass, ytterbium fluoride, zirconia, titania, and colloidal silica. Of those, particles formed of a silica-titanium group element oxide-based composite oxide are preferably used because their refractive index is easily adjusted.

Herein, the term "silica-titanium group element oxide-based composite oxide particles" means particles of a composite oxide of silica and an oxide of a titanium group element (element belonging to Group 4 of the periodic table), and the refractive index of the particles for a sodium d-line at 25°C can be changed within the range of from about 1.45 to about 1.58 in accordance with the content of the silica content thereof. Specific examples of the silica-titanium group element oxide-based composite oxide particles may include silica-titania, silica-zirconia, and silica-titania-zirconia particles. Of those, silica-zirconia particles are preferably used. Although a composite ratio in the silica-zirconia particles is not particularly limited, particles in each of which a silica content is from 70 mol% to 95 mol%, and a titanium group element oxide content is from 5 mol% to 30 mol% are preferred from the viewpoints of imparting sufficient X-ray opacity and setting the refractive index within a suitable range to be described later. Those silica-titanium group element oxide-based composite oxide particles may be composited with a metal oxide except silica and the titanium group element oxide as long as the amount of the metal oxide is small. Specifically, an alkali metal oxide, such as sodium oxide or lithium oxide, may be incorporated at 10 mol% or less.

Although a method of producing such silica-titanium group element oxide-based composite oxide particles is not particularly limited, to obtain a spherical filler, for example, the following so-called sol-gel method is suitably adopted: a mixed solution containing a hydrolyzable organosilicon compound and a hydrolyzable organic titanium group metal compound is added to an alkaline solvent; and the mixture is hydrolyzed to precipitate a reaction product. Inorganic spherical particles formed of such silica-titanium group element oxide-based composite oxide are preferably subjected to surface treatment with a silane coupling agent, such as a $\gamma$-methacryloyloxyalkyltrimethoxysilane or hexamethyldisilazane.

(2-4) Relationship between Refractive Index of Cured Body of Polymerizable Monomer and Refractive Index of Inorganic Spherical Particles

In each of the existing structural color-based dental curable composition and the dental curable composition obtained by the present dental curable composition production method, when the refractive index of "the cured body of the polymerizable monomer" (specifically, a cured product of a composition formed of the polymerizable monomer and a small amount of the polymerization initiator), which corresponds to the resin matrix in the cured body of the dental curable composition, for a sodium d-line at 25°C is represented by $n_{(MX)}$, and the refractive indices of the inorganic spherical particles for forming the respective G-PID$_m$s for the sodium d-line at 25°C are each represented by n$_{(G\text{-}PIDm)}$, the following relationship needs to be valid for any one of the n$_{(G\text{-}PIDm)}$s.

$$n_{(MX)} < n_{(G\text{-}PIDm)}$$

In the case where the above-mentioned relationship is not satisfied, even when a structural color is expressed, light having a short wavelength is liable to be scattered in the resin matrix in the cured body of the dental curable composition, and hence it becomes difficult to observe the expressed structural color. From the viewpoints of the visibility and clarity of the expressed structural color, and color tone compatibility when the composition is used as a dental filling restoration material, $\Delta$n serving as a difference between the n$_{(G\text{-}PIDm)}$ and the n$_{(MX)}$ is preferably 0.001 or more and 0.1 or less, more preferably 0.002 or more and 0.1 or less, most preferably 0.005 or more and 0.05 or less.

As described above, when the refractive index of a polymerizable monomer composition for a sodium d-line at 25°C is set within the range of from 1.38 to 1.55, the refractive index (n$_{(MX)}$) of the cured body serving as the above-mentioned resin matrix can be set within the range of from 1.40 to 1.57. In addition, as described above, the refractive index (n$_{(G\text{-}PIDm)}$) of the silica-titanium group element oxide-based composite oxide can be changed within the range of from about 1.45 to about 1.58 by changing the silica content thereof. Accordingly, the $\Delta$n may be easily set within the suitable ranges by, for example, utilizing those relationships.

(2-5) Preferred Blending Mode of G-PID

At least part of the one or the plurality of groups of spherical particles having identical diameters are preferably blended as an organic-inorganic composite filler, which includes one kind of group of spherical particles having an identical diameter, and a resin whose refractive index for a sodium d-line at 25°C is smaller than the refractive index of the inorganic spherical particles for forming the one kind of group of spherical particles having an identical diameter at 25°C, and which is free of a group of spherical particles having an identical diameter except the above-mentioned one kind of group of spherical particles having an identical diameter (i.e., an organic-inorganic composite filler including only a single G-PID), because the above-mentioned short-distance ordered structure can be obtained more simply and more reliably.

The term "organic-inorganic composite filler" as used herein means a filler formed of: powder formed of such a composite that an inorganic filler is dispersed in an (organic) resin matrix; or an aggregate in which the primary particles of the inorganic filler are bonded to each other by an (organic) resin. For example, when the one or the plurality of the "groups of spherical particles having identical diameters" include three kinds of G-PIDs having different average primary particle diameters, that is, the G-PID$_1$, the G-PID$_2$, and the G-PID$_3$, the above-mentioned preferred aspect is such that the entirety or part of at least one kind of the G-PIDs is blended as an "organic-inorganic composite filler including only a single G-PID." If the entirety of the G-PID$_1$ is blended as an organic-inorganic composite filler including only the G-PID$_1$ (composite filler 1) into the curable composition, the structural color of the G-PID$_1$ is reliably expressed even in a composite material obtained by curing the curable composition because only the G-PID$_1$ is incorporated into the composite filler 1, and hence a short-distance ordered structure that may express the structural color of the G-PID$_1$ is achieved.

From the viewpoint that such effect can be expected, and the viscosity of the curable composition is easily adjusted, it is preferred that 10% to 90%, more preferably 20% to 80%, still more preferably 30% to 70% of each of the G-PIDs be blended as an "organic-inorganic composite filler including only a single G-PID." At this time, the refractive index n'$_{(F\text{-}MX)}$ of the resin matrix of the organic-inorganic composite filler for a sodium d-line at 25°C needs to be smaller than the refractive index (n$_{(G\text{-}PIDm)}$) of the inorganic spherical particles for the sodium d-line at 25°C. In addition, as in the n$_{(MX)}$, $\Delta$n' serving as a difference between the n$_{(G\text{-}PIDm)}$ and the n'$_{(F\text{-}MX)}$ preferably falls within the same ranges as those of the $\Delta$n. Further, the absolute value |n$_{(MX)}$-n'$_{(F\text{-}MX)}$| of a difference between the refractive index n$_{(MX)}$ of the cured body of the polymerizable monomer in the structural color-based dental curable composition of the present invention (specifically, the cured product of the composition formed of the polymerizable monomer and a small amount of the polymerization initiator) for the sodium d-line at 25°C and the n'$_{(F\text{-}MX)}$ needs to be from 0 to 0.1 because the transparency of the cured body of the dental curable composition is hardly reduced.

In addition, the amount of the inorganic spherical particles to be blended into the organic-inorganic composite filler is preferably from 30 mass% to 95 mass%, particularly preferably from 40 mass% to 90 mass%. In addition, although the average particle diameter of the particles is not particularly limited, a median diameter determined on the basis of the results of measurement by a laser diffraction/scattering method with a particle size distribution meter is preferably from 2 μm to 100 μm, more preferably from 5 μm to 50 μm, still more preferably from 5 μm to 30 μm from the viewpoint of improving the mechanical strength of the composite material and the operability of the curable composition.

(2-6) Amount of G-PID to be used

The total amount of the G-PID to be used is from 10 parts by mass to 1,500 parts by mass with respect to 100 parts by mass of the polymerizable monomer. The total amount is preferably from 50 parts by mass to 1,500 parts by mass, more preferably from 100 parts by mass to 1,500 parts by mass because the composite material to be obtained has moderate transparency and a high structural color-expressing effect. When such composition includes a plurality of kinds of G-PIDs, the content of each of the G-PIDs only needs to be appropriately set to such an amount that the total content falls within the above-mentioned ranges in consideration of the color tone of a structural color based on each of the G-PIDs and a color tone desired in the composite material.

(2-7) Polymerization Initiator

As the polymerization initiator, a chemical polymerization initiator and/or a photopolymerization initiator is preferably used as the polymerization initiator in the dental curable composition of the present invention because such composition is often cured in an oral cavity. The photopolymerization initiator is more preferably used because there is no need to perform a mixing operation. Although those polymerization initiators may each be used alone, two or more kinds thereof may be used as a mixture. Although an effective amount only needs to be selected as the blending amount of the polymerization initiator in accordance with purposes, the polymerization initiator is typically used at a ratio of from 0.01 part by mass to 10 parts by mass, more preferably at a ratio of from 0.1 part by mass to 5 parts by mass with respect to 100 parts by mass of the polymerizable monomer.

Examples of the photopolymerization initiator that may be suitably used may include benzoin alkyl ethers, benzyl ketals, benzophenones, $\alpha$-diketones, thioxanthone compounds, and bisacyl phosphine oxides. A reducing agent is often added to the photopolymerization initiator. Examples of the reducing agent include aromatic amines, aliphatic amines, aldehydes, and sulfur-containing compounds. Further, a trihalomethyl triazine compound, an aryliodonium salt, or the like may be added thereto as required.

In addition, examples of the thermal polymerization initiator that may be suitably used may include: peroxides, such as benzoyl peroxide, p-chlorobenzoyl peroxide, tert-butyl peroxy-2-ethylhexanoate, tert-butyl peroxydicarbonate, and diisopropyl peroxydicarbonate; azo compounds such as azobisisobutyronitrile; boron compounds, such as tributylborane, partially oxidized tributylborane, sodium tetraphenylborate, sodium tetrakis(p-fluorophenyl)borate, and a tetraphenylboric acid triethanolamine salt; barbituric acids, such as 5-butylbarbituric acid and 1-benzyl-5-phenylbarbituric acid; and sulfinic acid salts, such as sodium benzenesulfinate and sodium p-toluenesulfinate.

(2-8) Specific X-ray Opaque Filler

The specific X-ray opaque filler has already been described, but the $|n_{(MX)}-n_{(F-MX)}|$ needs to be from 0 to 0.1. When the condition is not satisfied, the transparency of the cured body of the structural color-based dental curable composition of the present invention significantly reduces. To satisfy the above-mentioned condition, in the specific X-ray opaque filler to be blended into the structural color-based dental curable composition of the present invention, a polymerizable monomer having composition identical or approximate to that of the polymerizable monomer in the structural color-based dental curable composition of the present invention is preferably used as a polymerizable monomer serving as a raw material for (the matrix resin of) the filler. The $|n_{(MX)}-n_{(F-MX)}|$ is more preferably from 0 to 0.05.

From the viewpoints of the X-ray opacity, transparency, and structural color expressivity of the cured body, the amount of the specific X-ray opaque filler to be used is from 1 part by mass to 100 parts by mass, preferably from 15 parts by mass to 75 parts by mass with respect to 100 parts by mass of the polymerizable monomer in terms of the total mass of the low crystalline rare earth metal fluoride particulate object. When the amount is less than 1 part by mass (lower limit value), sufficient X-ray opacity is not obtained, and when the amount is more than 100 parts by mass (upper limit), the transparency or the structural color expressivity significantly reduces.

(2-9) Other Filler

The dental curable composition may be blended with a group of superfine particles (G-SFP), which is a particle aggregate formed of inorganic particles having an average primary particle diameter of less than 100 nm, for the purpose of, for example, adjusting the viscosity of the dental curable composition to be obtained or adjusting the transparency of the cured body thereof. However, the average primary particle diameter of the G-SFP needs to be smaller than the average primary particle diameter ($d_1$) of the $G-PID_1$ having the smallest average primary particle diameter out of the G-PIDs to be blended by 25 nm or more. When such condition is not satisfied, the dispersed state of the inorganic spherical particles is adversely affected, and hence their structural color is hardly expressed. The shape of each of the inorganic particles for forming the G-SFP is not particularly limited, and may be an irregular shape or a spherical shape. In addition, the lower limit of the average primary particle diameter is typically 2 nm. The average primary particle diameter of the G-SFP is preferably from 3 nm to 75 nm, more preferably from 5 nm to 50 nm because an influence on the expression of the structural color is small. In addition, the average primary particle diameter of the G-SFP is smaller than the average primary particle diameter ($d_1$) of the G-PID$_1$ by preferably 30 nm or more, more preferably 40 nm or more because of the same reason. The same material as that for the inorganic spherical particles may be used as a material for the inorganic particles for forming the G-SFP without any particular limitation. In addition, the inorganic particles may be subjected to surface treatment with a silane coupling agent as in the inorganic spherical particles. A suitable aspect thereof is basically the same as that of the inorganic spherical particles except for their average primary particle diameter and shapes. The content of the G-SFP only needs to be appropriately determined in consideration of, for example, the viscosity of the curable composition and the transparency (or contrast ratio serving as an indicator thereof) of the cured body. However, the content is typically from 0.1 part by mass to 50 parts by mass, preferably from 0.2 part by mass to 30 parts by mass with respect to 100 parts by mass of the polymerizable monomer.

In addition, the dental curable composition may include rare earth metal fluoride particles except the rare earth metal fluoride particles to be blended as the specific X-ray opaque filler to the extent that the effect of the present invention is not remarkably inhibited. The blending amount of such rare earth metal fluoride particles is set to preferably 5 parts by mass or less, particularly preferably from 0 parts by mass to 3 parts by mass with respect to 100 parts by mass of the polymerizable monomer. It is particularly preferred that the blending amount of the crystalline rare earth metal fluoride particles having a full width at half maximum of less than 0.3° be set to from 0 parts by mass to 0.5 part by mass or less. When the specific X-ray opaque filler and non-composited particles having a full width at half maximum of 0.3° or more are used in combination, the total blending amount of the crystalline rare earth metal fluoride particles having a full width at half maximum of 0.3° or more in the specific X-ray opaque filler and the non-composited particles is set to preferably from 0 parts by mass to 100 parts by mass, particularly preferably from 0 parts by mass to 50 parts by mass with respect to 100 parts by mass of the polymerizable monomer.

(2-10) Other Additive and the Like

The dental curable composition may be blended with any other additive, such as a polymerization inhibitor or a UV absorber, to the extent that the effect of the present invention is not inhibited. As described above, the cured body of the dental curable composition to be obtained expresses its structural color even when a coloring substance such as a pigment is not used. Accordingly, there is no need to blend a pigment whose color may change with the lapse of time. However, the blending itself of the pigment is not denied, and the pigment may be blended to such an extent as not to inhibit colored light caused by the interference of a spherical filler. Specifically, the pigment may be blended in an amount of from about 0.0005 part by mass to about 0.5 part by mass, preferably from about 0.001 part by mass to about 0.3 part by mass with respect to 100 parts by mass of the polymerizable monomer.

(3) Mixing Method

The present dental curable composition production method includes a mixing step of weighing and mixing the predetermined amounts of all the components serving as raw materials. In the mixing step, the dental curable composition is preferably prepared by a method including performing the mixing by adopting the mixing conditions under which the following is recognized for a mixture obtained in the step: the dispersed state of the inorganic particles in a cured body obtained by curing the mixture satisfies the following condition (I) and condition (II).

[Conditions that Dispersed State should satisfy]

(I) In a radial distribution function graph showing a relationship between a normalized dimensionless number ($r/r_0$), which is obtained by dividing the distance "r" from the center of the arbitrary inorganic spherical particle dispersed in the cured body by the average particle diameter $r_0$ of the entirety of the inorganic spherical particles dispersed in the cured body, and the radial distribution function g(r) corresponding to the "r" at the time, the graph having an x-axis indicating the ratio "$r/r_0$" and a y-axis indicating the g(r), a nearest-neighbor particle distance $r_1$ defined as the "r" corresponding to the peak top of a peak that is closest to an origin out of peaks appearing in the radial distribution function graph is a value once or more and twice or less as large as the average particle diameter $r_0$ of the entirety of the inorganic spherical particles dispersed in the cured body of the mixture.

(II) When the "r" corresponding to the peak top of a peak that is second closest to the origin out of the peaks appearing in the radial distribution function graph is defined as a second-nearest-neighbor particle distance $r_2$, the local minimum value of the radial distribution function g(r) between the nearest-neighbor particle distance $r_1$ and the second-nearest-

neighbor particle distance $r_2$ is a value of 0.56 or more and 1.10 or less.

Herein, the radial distribution function g(r) is well known as a function for determining a probability that any other particle is present at a point distant from an arbitrary particle by a distance "r", and the function is defined by the following equation (1).

$$g(r)=\{1/<\rho>\}\times\{dn/da\}\cdots(1)$$

In the equation (1), $<\rho>$ represents the average particle density of the particles in a plane, dn represents the number of the particles present in a region between two circles, which are centered around the arbitrary particle in the plane and have radii of "r" and r+dr, and da represents the area of the above-mentioned region, that is, $2\pi r \cdot dr$.

The radial distribution function g(r) is generally represented by a radial distribution function graph whose x-axis (distance axis) indicates the distance "r", and whose y-axis (axis of ordinate) indicates the value of the g(r) {the result of calculation by the equation (1)} at the "r", or a radial distribution function graph whose distance axis indicates a normalized dimensionless number, which is obtained by dividing the "r" by the average particle diameter of the particles, and whose y-axis (axis of ordinate) indicates the value of the g(r) (the result of calculation by the equation) at the "r" corresponding to a value on the x-axis.

The g(r) calculated by the equation (1) on the basis of the $<\rho>$ and the dn, which are determined as follows, and the da ($=2\pi r \cdot dr$) in accordance with the value of the dr adopted at the time of the determination of the above-mentioned dn is preferably adopted because the $<\rho>$ and the dn are easily and reliably identified: a plane inside the cured body of the mixture is adopted as an observation plane, and the $<\rho>$ and the dn are determined on the basis of a scanning electron microscope image of the plane. The $<\rho>$, the dn, and the da may be determined as described below. First, the mixture is cured, and a plane (observation plane) in which the dispersed state of the inorganic spherical particles in the resultant cured body can be observed is exposed to the surface of the cured body by a method such as the abrasion of the surface. Next, the observation plane is observed with a scanning electron microscope, and the microscope image of a region including at least 500 inorganic spherical particles in the plane is obtained. After that, the coordinates of the inorganic spherical particles in the region are determined by analyzing the resultant scanning electron microscope image with image analysis software (e.g., "Simple Digitizer ver. 3.2" free software). One pair of coordinates of an arbitrary inorganic spherical particle is selected from the resultant coordinate data, and the following circle is drawn around the selected inorganic spherical particle serving as a center: such a distance "r" from the center that at least 200 inorganic spherical particles are included in the circle is defined as the radius of the circle. The average particle density $<\rho>$ (unit: particles/cm$^2$) may be determined by counting the number of the inorganic spherical particles in the circle.

In addition, with regard to the dn, the dn may be determined as follows: when the average particle diameter of the inorganic spherical particles is represented by $r_0$, the dr whose length is a value of from about $r_0/100$ to about $r_0/10$ is set; one randomly selected inorganic spherical particle is adopted as a central particle; and the number of the inorganic spherical particles in a region between a circle whose distance "r" from the center of the particle is adopted as its radius and a circle having a radius of r+dr, which is concentric with the circle, is counted. Further, the da that is the area of the region between the two circles is determined as $2\pi r \cdot dr$ on the basis of the length of the actually set dr.

In addition, a graph showing the relationship between the normalized dimensionless number ($r/r_0$), which is indicated by its x-axis, and the radial distribution function g(r) corresponding to the "r" at the time, which is indicated by its y-axis, the ratio "$r/r_0$" and the g(r) being defined as follows, serves as a radial distribution function graph: the distance "r" from the center of the arbitrary inorganic spherical particle dispersed in the cured body is divided by the average particle diameter $r_0$ of the entirety of the inorganic spherical particles dispersed in a composite material to provide the ratio "$r/r_0$"; and the probability that any other inorganic spherical particle is present at the point distant from the center of the above-mentioned arbitrary inorganic spherical particle by the distance "r" is represented by the g(r). From the viewpoint that it becomes easier to maintain the short-distance orderliness of the inorganic spherical particles, and to express their structural color, the ratio "$r_1/r_0$" is from 1.0 to 2.0, preferably from 1.0 to 1.5. In addition, from the viewpoint that it becomes easier to express the structural color, and to obtain the color tone compatibility as a dental filling material, the local minimum value of the radial distribution function g(r) between the nearest-neighbor particle distance $r_1$ and the second-nearest-neighbor particle distance $r_2$ is a value of from 0.56 to 1.10, preferably a value of from 0.56 to 1.00.

To facilitate the satisfaction of those conditions, in the mixing step, the inorganic spherical particles (G-PID) are mixed as an organic-inorganic composite filler having a particle diameter of preferably from 5 μm to 50 μm, more preferably from 5 μm to 30 μm, or are mixed as an aggregated particle having a particle diameter of preferably from 5 μm to 200 μm, more preferably from 10 μm to 100 μm. In addition, for example, deaeration treatment is preferably performed to prevent bubbles from remaining at least after the mixing because the inclusion of the bubbles during the mixing not only makes it difficult to satisfy the above-mentioned conditions but also serves as a defect of the composite material. A method including performing deaeration under reduced pressure is preferably adopted as a deaeration method because bubbles can be removed even from a high-viscosity composition in a short time period. When the inorganic spherical particles are mixed while attention is paid to such point, the performance of sufficient stirring satisfies the above-mentioned conditions in principle. However, even when it is visually judged that the mixture is brought into a uniform state, from the viewpoint of satisfying the above-mentioned conditions, the stirring may be insufficient, and hence it is difficult to find out its endpoint. Accordingly, the mixing step is preferably performed after the endpoint has been found out by the above-mentioned method (a) or (b), or while the endpoint is found out by such method:

(a) a method including adopting the same mixing conditions as mixing conditions satisfying the condition (I) and the condition (II), the mixing conditions being determined by: mixing materials for a curable composition, which has the same composition, or substantially the same composition, as that of the curable composition to be actually produced, in advance while changing mixing conditions in a plurality of ways; and examining the radial distribution function g(r) in each of the cured bodies of mixtures obtained at the time of the mixing under the respective mixing conditions; and (b) a method including: sampling part of the mixture obtained during and/or after the completion of the mixing step; recognizing whether or not the dispersed state of the inorganic particles in a cured body of the sampled mixture satisfies the condition (I) and the condition (II); and continuing the mixing until these conditions are satisfied.

4. Method of curing Dental Curable Composition obtained by the Present Dental Curable Composition Production Method

Known polymerization means only needs to be appropriately adopted as means for curing the dental curable composition obtained by the present dental curable composition production method in accordance with the polymerization initiation mechanism of the polymerization initiator used. Specifically, for example, irradiation with light from a light source, such as a carbon arc, a xenon lamp, a metal halide lamp, a tungsten lump, a fluorescent lamp, sunlight, a helium cadmium laser, or an argon laser, heating with a heat curing unit or the like, or a method including a combination thereof is used without any limitation. When polymerization is performed through the irradiation with light, an irradiation time varies depending on the wavelength and intensity of a light source, and the shape and material of the cured body, and hence only needs to be determined in advance through a preliminary experiment. When the curable composition of this embodiment is used in dental applications, it is generally preferred that the blending ratios of its various components be adjusted so that the irradiation time may fall within the range of from about 5 seconds to about 60 seconds.

Examples

The present invention is specifically described below by way of Examples for Reference (Reference Examples and Reference Comparative Examples), and Examples and Comparative Examples, but the present invention is not limited to these examples only.

1. Substance Names and Abbreviations thereof

(1) Polymerizable Monomers

UDMA: 1,6-bis(methacrylethyloxycarbonylamino)-2,2,4-trimethylhexane
3G: triethylene glycol dimethacrylate

(2) Polymerization Initiators

CQ: camphorquinone (manufactured by Tokyo Chemical Industry Co., Ltd.)
DMBE: ethyl dimethylbenzoate (manufactured by Tokyo Chemical Industry Co., Ltd.)
AIBN: azobisisobutyronitrile (manufactured by Tokyo Chemical Industry Co., Ltd.)

(3) Polymerizable Monomer Composition (Monomer Composition)

M1: A liquid composition prepared by stirring and mixing the mixture of UDMA (80 parts by mass), 3G (20 parts by mass), CQ (0.2 part by mass), and DMBE (0.35 part by mass) for 6 hours

M2: A liquid composition prepared by stirring and mixing the mixture of UDMA (80 parts by mass), 3G (20 parts by mass), and AIBN (1 part by mass) for 6 hours

The refractive indices of the curable components M1 and M2 before and after their curing measured in accordance with a method to be described later are shown in Table 1.

Table 1

| Monomer composition | Composition (part(s) by mass) | Refractive index | |
| --- | --- | --- | --- |
| | | Before curing | After curing |
| M1 | UDMA(80)/3G(20)/CQ(0.2)/DMBE(0.35) | 1.478 | 1.509 |
| M2 | UDMA(80)/3G(20)/AIBN(1) | 1.478 | 1.509 |

(4) Highly Crystalline Rare Earth Metal Fluoride Particulate Object

YbF3-40: Ytterbium fluoride having the following various properties (manufactured by Sukgyung AT Co., Ltd.): corresponding to F-1 to be described later

·Average primary particle diameter: 40 nm
·Average secondary particle diameter: 0.6 $\mu$m
·Refractive index: 1.55
·Full width at half maximum of a peak derived from a (111) plane (peak observed around $2\theta=28°$) serving as the peak of YbF$_3$ having the maximum intensity in its X-ray diffraction measurement: 0.17°

Those physical property values are values measured in accordance with methods to be described later.

(5) Group of Spherical Particles having Identical Diameter (G-PID)

PF-1: A product obtained by subjecting silica-zirconia-based spherical particles having the following various properties (composition: SiO$_2$/ZrO$_2$/Na$_2$O=89.8/9.0/1.2 (mol%)) to surface treatment with a silane coupling agent (3-(trimethoxysilyl)propyl-3-(methacryloyloxy)propyltrimethoxysilane methacrylate (manufactured by Tokyo Chemical Industry Co., Ltd.))

·Average primary particle diameter: 260 nm
·Refractive index: 1.515
·Evenness: 0.90
·5% Particle percentage content: 92%

The term "evenness" as used herein means the ratio (D2/D1) of a particle diameter D2 in a direction perpendicular to the maximum diameter D1 of each of the spherical particles to the maximum diameter D1. In addition, the average primary particle diameter and the refractive index are values measured in accordance with methods to be described later.

2. Methods of measuring and evaluating the Physical Property Values of Raw Materials

(1) Refractive Index Measurement

(1-1) Refractive Indices of Monomer Compositions M1 and M2 (before Curing)

The refractive indices of the M1 and the M2 (before their curing) were each measured with an Abbe refractometer (DR-A1-Plus, manufactured by Atago Co., Ltd.) as a refractive index for a sodium d-line at 25°C.

(1-2) Refractive Indices of Monomer Compositions M1 and M2 (after Curing)

The monomer compositions were each loaded into a through-hole (diameter: 7 mm, through-hole length: 0.5 mm) formed in a mold, and then opening portions at both sides of the through-hole were each sealed with a polyester film while the film was brought into pressure contact therewith. After that, with regard to the M1, the curable component M1 loaded into the through-hole was cured by being irradiated with light through use of a halogen-type dental light irradiator (Demetron LC, manufactured by Sybron) at a light amount of 500 mW/cm$^2$ for 30 seconds. In addition, the M2 was cured by being heated with a nitrogen pressure heat polymerizer POLYNER (manufactured by Towa Giken K.K.) under nitrogen pressure at 100°C for 30 minutes instead of being irradiated with light. After that, the refractive indices of the cured products of the M1 and the M2 removed from the mold were measured by the same procedure as that in the section (1-1).

(1-3) Refractive Indices of Rare Earth Metal Fluoride Particles and G-PID

In a constant-temperature room at 25°C, 1 g of powder to be measured was suspended in 50 mL of anhydrous toluene in a 100 mL sample bottle. While the resultant suspension was stirred with a stirrer, 1-bromotoluene was dropped thereinto little by little. The refractive index of the suspension at the time point when the suspension became most transparent was measured by the same procedure as that in the section (1-1).

(2) Average Primary Particle Diameters of Rare Earth Metal Fluoride Particles and G-PID

The average primary particle diameter of each of the rare earth metal fluoride particles and the G-PID was determined by the following procedure with a scanning electron microscope. First, a measurement sample was prepared by fixing the rare earth metal fluoride particles or the G-PID onto a sample stage with a carbon paste and then performing conductive treatment (platinum vapor deposition). Next, the measurement sample was observed with an electron microscope (JSM-7800F PRIME, manufactured by JEOL Ltd.) at a magnification of 100,000. The average particle diameter of 100 primary particles in the resultant observation image was determined as the average primary particle diameter. In addition, the average evenness of the G-PID was determined on the basis of primary particles (100 particles) in the resultant observation image.

(3) Measurement of Average Secondary Particle Diameter of Rare Earth Metal Fluoride Particles

The average secondary particle diameter of the rare earth metal fluoride particles was determined by the following procedure through particle size distribution measurement. First, a suspension in which 0.1 g of powder (rare earth metal fluoride particles) was suspended in 10 mL of ion-exchanged water was prepared. Next, under a state in which the suspension was irradiated with an ultrasonic wave, particle size distribution measurement was performed with a particle size distribution analyzer (LS13-320, manufactured by Beckman Coulter, Inc.) to provide a volume-based particle size distribution. Then, a particle diameter (D50v value) corresponding to the cumulative 50% of the volume-based particle size distribution from small diameters was defined as the average secondary particle diameter of the rare earth metal fluoride particles.

(4) Identification of Average Primary Particle Diameter of G-PID through Particle Size Distribution Measurement

The number-based particle size distribution of the G-PID was determined by the following procedure through particle size distribution measurement. First, a suspension in which 0.1 g of powder (G-PID) was suspended in 10 mL of ion-exchanged water was prepared. Next, under a state in which the suspension was irradiated with an ultrasonic wave, particle size distribution measurement was performed with a particle size distribution analyzer (LS13-320, manufactured by Beckman Coulter, Inc.) to provide a number-based particle size distribution. Then, a particle diameter (D50p value) corresponding to the cumulative 50% of the number-based particle size distribution from smaller diameters was determined. As a result, the particle diameter coincided well with the average primary particle diameter determined by electron microscope observation.

3. Examples for Reference (Reference Examples 1 to 13 and Reference Comparative Examples 1 to 5)

To observe the curing of the specific X-ray opaque filler, the specific X-ray opaque filler and an X-ray opaque filler that did not correspond thereto were each prepared in a small amount (for which there was no need to use the present X-ray opaque filler production method) by using the above-mentioned raw materials. The evaluations of those X-ray opaque fillers, and the preparation and evaluations of curable compositions blended with those X-ray opaque fillers were performed.

3-1. Production of X-ray Opaque Filler

(1) Mechanochemical Treatment of Crystalline Rare Earth Metal Fluoride Particulate Object

855 Grams of ion-exchanged water and 45 g of YbF3-40 (highly crystalline rare earth metal fluoride particulate object) were mixed to prepare a slurry. Subsequently, the slurry was subjected to dispersion treatment through use of a circulating wet bead mill SC50 (manufactured by Mitsui Mining Co., Ltd.) and 100 g of zirconia beads (diameter: 0.3 mm) as media at a number of revolutions of 3,000 rpm. Conditions for the dispersion treatment at this time are shown in Table 2.
The slurry after the dispersion treatment was concentrated with a rotary evaporator under the condition of a bath temperature of 50°C to provide a particulate object. The particulate object was dried in a vacuum at 80°C for 15 hours to provide a mechanochemically treated rare earth metal fluoride particulate object. The average primary particle diameters of rare earth metal fluoride particles (the very crystalline rare earth metal fluoride particulate object YbF3-40) F-1 corresponding to a dispersion treatment time of 0 minutes and mechanochemically treated rare earth metal fluoride particulate objects F-3 to F-6 were measured by the method described in the section 2.(2), and the full widths at half maximum S thereof were measured by the following method. The results are collectively shown in Table 2.
Method of measuring Full Width at Half Maximum S: A measurement sample was prepared by removing coarse particles from a particulate object serving as an object of X-ray diffraction measurement with a sieve. Next, the measurement sample was loaded into the sample stage of an X-ray diffractometer (SmartLab, manufactured by Rigaku Corporation), and was subjected to the X-ray diffraction measurement to provide an X-ray diffraction pattern (chart) whose axis of abscissa indicated $2\theta$ (°), and whose axis of ordinate indicated a diffraction intensity. Here, a CuK$\alpha$ ray was used as the X-ray of the X-ray diffraction measurement. The rare earth metal fluoride is YbF$_3$, and hence the full width at half maximum of a peak resulting from its (111) plane having the maximum intensity (peak observed around $2\theta=28°$) was determined. When the measurement powder is changed to an X-ray opaque filler to be described later, the full width at half maximum C of the X-ray opaque filler may be determined by the same method.

Table 2

| Rare earth metal fluoride particles | Mechanochemical treatment time [min.] | Average primary particle diameter [nm] | Maximum peak: (111) plane | |
| --- | --- | --- | --- | --- |
| | | | $2\theta$ [°] | Full width at half maximum S [°] |
| F-1 | 0 | 46 | 28.0 | 0.17 |
| F-2 | 90 | 39 | 28.0 | 0.32 |
| F-3 | 180 | 44 | 27.9 | 0.43 |
| F-4 | 240 | 33 | 27.9 | 0.51 |
| F-5 | 300 | 28 | 27.8 | 0.59 |
| F-6 | 600 | 16 | 27.9 | 0.77 |

(2) Preparation of Curable Raw Material Composition, and Curing and Pulverization Treatment thereof

25 Parts by mass of the monomer composition M2 and 75 parts by mass of the rare earth metal fluoride particulate object F-3 were weighed, and were mixed with an agate mortar to prepare a paste-like curable raw material composition. The curable raw material composition thus obtained was thermally cured by being heated with a nitrogen pressure heat polymerizer POLYNER (manufactured by Towa Giken K.K.) under nitrogen pressure at 100°C for 30 minutes. Thus, a cured product was obtained. The resultant cured body and zirconia balls (diameter: 25 mm) were loaded into a zirconia-made pot, and were subjected to rotary pulverization treatment for 60 minutes to provide a pulverized product of the cured product. Coarse particles were removed from the pulverized product with a stainless steel-made sieve having an aperture of 45 $\mu$m. Thus, an X-ray opaque filler CF-1 (serving as a specific X-ray opaque filler) was obtained. The average particle diameter and full width at half maximum C of the X-ray opaque filler CF-1 were measured. The results are shown in Table 3. Fillers CF-1 to CF-4 and CF-1a to CF-1i other than a filler RCF-1 obtained in Production Example 5 are specific X-ray opaque fillers.
In addition, the average particle diameter of the X-ray opaque filler was determined by the following procedure through particle size distribution measurement. That is, a suspension in which 0.1 g of powder (X-ray opaque filler) was suspended in 10 mL of ethanol was prepared first. Next, under a state in which the suspension was irradiated with an ultrasonic wave, particle size distribution measurement was performed with a particle size distribution analyzer (LS13-320, manufactured by Beckman Coulter, Inc.) to provide a volume-based particle size distribution. Then, a particle diameter (D50v value) corresponding to the cumulative 50% of the volume-based particle size distribution from small diameters was defined as the average particle diameter of the X-ray opaque filler. In addition, the full width at half maximum C was measured by the same method as that of the full width at half maximum S. The results are collectively shown in Table 3.

Table 3

| Production Example No. | Raw material composition | | | X-ray opaque filler | | |
|---|---|---|---|---|---|---|
| | Monomer composition | Rare earth metal fluoride particles | | | | |
| | | Abbreviation | Full width at half maximum S [°] | Abbreviation | Average particle diameter [μm] | Full width at half maximum C [°] |
| 1 | M2 | F-3 | 0.43 | CF-1 | 24.9 | 0.40 |
| 2 | | F-4 | 0.51 | CF-2 | 24.7 | 0.53 |
| 3 | | F-5 | 0.59 | CF-3 | 26.3 | 0.58 |
| 4 | | F-6 | 0.77 | CF-4 | 25.2 | 0.75 |
| 5 | | F-1 | 0.17 | RCF-1 | 24.7 | 0.19 |
| 6 | | F-3 | 0.43 | CF-1a | 4.8 | 0.41 |
| 7 | | | | CF-1b | 7.6 | 0.40 |
| 8 | | | | CF-1c | 9.4 | 0.42 |
| 9 | | | | CF-1d | 10.5 | 0.42 |
| 10 | | | | CF-1e | 14.8 | 0.40 |
| 11 | | | | CF-1f | 17.5 | 0.41 |
| 12 | | | | CF-1g | 54.4 | 0.41 |
| 13 | | | | CF-1h | 86.4 | 0.41 |
| 14 | | | | CF-1i | 102.3 | 0.42 |

3-2. Preparation of Curable Composition

An X-ray opaque filler (16 parts by mass) and the G-PID "PF-1" (64 parts by mass) were added to the monomer composition M1 (20 parts by mass), and then the materials were mixed with an agate mortar to provide a mixture. Further, bubbles were removed by deaerating the mixture in a vacuum. Thus, a paste-like curable composition was obtained. At the time of the preparation of the curable composition, curable compositions of Reference Examples 1 to 13 and Reference Comparative Examples 1 to 5 were each prepared by changing the kind of the X-ray opaque filler as shown in Table 4.

3-3. Evaluation of Cured Body

The X-ray radiography contrast, transparency, spectral reflectance ratio, and bending strength of each of the cured bodies of the respective curable compositions obtained by the above-mentioned method were measured, and the dispersed state (radial distribution function) of the inorganic spherical particles in the cured body was evaluated. Methods of measuring the above-mentioned respective physical properties and a method for the evaluation are described below. In addition, the results of the evaluation are shown in Table 5.

(1) Method of measuring X-ray Radiography Contrast

The X-ray radiography contrast of each of the cured bodies of the curable compositions was measured by the following procedure. First, the curable composition was loaded into a through-hole (having a diameter of 15 mm and a through-hole length of 1.0 mm) formed in a polytetrafluoroethylene-made mold, and then opening portions at both the ends of the through-hole were each sealed with a polypropylene film while the film was brought into pressure contact therewith. Next, photoirradiation was performed under a state in which a dental light irradiator (TOKUSO POWER LIGHT, manufactured by Tokuyama Dental Corporation) was arranged so as to be brought into close contact with the surface of the polypropylene film sealing each of the opening portions of the through-hole. The photoirradiation was performed at the following positions: five sites for one of the opening portion sides of the through-hole (one site in the central portion of the through-hole and four sites in a portion inside the outer edge of the through-hole); and five sites for the other opening portion side thereof (one site in the central portion of the through-hole and four sites in a portion inside the outer edge of the through-hole). Then, the photoirradiation was performed at each of the photoirradiation positions for 20 seconds to provide the cured body. The thickness of the resultant cured body was identified with a micrometer. Then, the cured body having a thickness in the range of 1.0 mm±0.1 mm was adopted as a test piece to be used in X-ray radiography contrast measurement.

Next, an X-ray film (super-high sensitivity dental X-ray film, manufactured by Eastman Kodak Company) was arranged on a lead sheet having a thickness of 2.0 mm, and then the test piece and an aluminum-made step wedge having five-stage thicknesses (thicknesses: 1.0±0.01 mm, 2.0±0.01 mm, 3.0±0.01 mm, 4.0±0.01 mm, and 5.0±0.01 mm) were further arranged on the X-ray film. Subsequently, an X-ray was applied from a position at a height of 40 cm from the surface of the X-ray film to the test piece and the step wedge by using an X-ray irradiation apparatus (PANPAS-E, manufactured by Yoshida). At this time, the irradiation was performed under the conditions of a tube voltage of 60 kVp and an irradiation time of 0.3 second. After that, the X-ray film was developed and printed on photographic paper. Next, the optical densities of a test piece image and a step wedge image printed on the photographic paper were measured. Subsequently, a calibration curve was produced on the basis of the five-stage thicknesses of the step wedge and the optical densities corresponding to these five-stage thicknesses, and the thickness of the step wedge (i.e., an aluminum material) at which the optical density of the test piece coincided with the optical density of the step wedge was determined on the basis of the calibration curve. Then, a value obtained by converting the determined thickness of the aluminum material into Al% with respect to an optical density (100 Al%) shown by a one-millimeter thick aluminum material was adopted as an evaluation indicator of the X-ray radiography contrast.

(2) Method of measuring Transparency

The transparency of each of the cured bodies of the curable compositions was measured by the following procedure. First, the curable composition was loaded into a through-hole (having a diameter of 0.7 cm and a through-hole length of 0.1 cm) formed in a polyacetal-made mold, and opening portions at both the ends of the through-hole were each sealed with a polypropylene film while the film was brought into pressure contact therewith. Next, a dental light irradiator (TOKUSO POWER LIGHT, manufactured by Tokuyama Dental Corporation) was arranged at a position distant from the opening surface of the hole by 0.5 cm, and photoirradiation was performed for 20 seconds to provide the cured body. The thickness of the resultant cured body was identified with a micrometer. Then, the cured body having a thickness in the range of 1.0 mm±0.1 mm was adopted as a cured body to be used in transparency evaluation. Subsequently, the Y-value (value related to brightness out of the tristimulus values of an XYZ colorimetric system specified in JIS Z8701) of the cured body under a black background and that under a white background were measured with a color-difference meter (SE 7700, manufactured by Nippon Denshoku Industries Co., Ltd.). Then, a contrast ratio C determined from the following equation was evaluated as an indicator of the transparency. A state in which the value of the contrast ratio C of a material is closer to 1 means that the material is more opaque, and a state in which the value is closer to 0 means that the material is more transparent.

.

$$\text{Equation: } C = Y_b / Y_w$$

Here, in the equation, Yb means a Y-value when the cured body is subjected to the measurement under the black background, and Yw means a Y-value when the cured body is subjected to the measurement under the white background.

(3) Method of measuring Spectral Reflectance Ratio (SR1/SR2 in Black Background)

The spectral reflectance ratios of the cured bodies of the curable compositions were determined by the following procedure. First, the spectral reflectance of the cured body used in the transparency evaluation in the wavelength range of from 380 nm to 780 nm was measured under a black background with a color-difference meter (SE 7700, manufactured by Nippon Denshoku Industries Co., Ltd.). Then, a spectral reflectance ratio R was determined on the basis of the following equation.

.

$$\text{Equation: } R=SR1/SR2$$

Here, in the equation, SR1 means the maximum value of the reflectance in a yellow to red wavelength region (from 600 nm to 750 nm), and SR2 means the maximum value of the reflectance in a blue wavelength region (from 400 nm to 500 nm).

(4) Method of measuring Bending Strength

The bending strength of each of the cured bodies was measured by the following procedure. First, the curable composition was loaded into a through-hole (having a length of 25 mm, a width of 2 mm, and a through-hole length of 2 mm) formed in a stainless steel-made mold, and then opening portions at both the ends of the through-hole were each sealed with a polypropylene film while the film was brought into pressure contact therewith. Next, photoirradiation was performed under a state in which a dental light irradiator (TOKUSO POWER LIGHT, manufactured by Tokuyama Dental Corporation) was arranged so as to be brought into close contact with the surface of the polypropylene film sealing each of the opening portions of the through-hole. The photoirradiation was performed at the following positions: three sites for one of the opening portion sides of the through-hole (one site at the center of the through-hole and two sites in a portion inside the outer edge at each of both the ends of the through-hole in its lengthwise direction); and three sites for the other opening portion side thereof (one site at the center of the through-hole and two sites in a portion inside the outer edge at each of both the ends of the through-hole in its lengthwise direction). Then, the photoirradiation was performed at each of the photoirradiation positions for 20 seconds to provide the cured body. The resultant cured body was adjusted with #1500 waterproof abrasive paper so as to have the following dimensional shape: a length of 25 mm±2 mm, a width of 2 mm±0.1 mm, and a thickness of 2 mm±0.1 mm. Then, the cured body whose dimensional shape had been adjusted was adopted as a test piece to be used in bending strength measurement. The test piece was mounted on a precision universal tester (AUTOGRAPH AG5000D, manufactured by Shimadzu Corporation), and its three-point bending fracture strength was measured under the conditions of an inter-fulcrum distance of 20 mm and a crosshead speed of 1 mm/min. Thus, a load-deflection curve was obtained. Then, the bending strength was determined on the basis of the following equation.

.

$$\text{Equation: } \sigma B=(3PS)/(2WB^2)$$

Here, symbols in the equation each have the following meaning: $\sigma B$ represents the bending strength (Pa), P represents a load (N) at the time of the fracture of the test piece, S represents the inter-fulcrum distance (m), W represents the width (m) of the test piece, and B represents the thickness (m) of the test piece.

(5) Method of evaluating Dispersed State (Radial Distribution Function) of Inorganic Spherical Particles

The dispersed state (radial distribution function) of the inorganic spherical particles in each of the curable compositions was evaluated by the following procedure. A cured body produced in the same manner as in the above-mentioned transparency evaluation was subjected to section milling with an ion milling apparatus (IM4000, manufactured by Hitachi, Ltd.) under the conditions of 2 kV and 20 minutes to provide an observation plane. After that, the cured body was fixed onto a sample stage with a carbon paste, and then the observation plane was subjected to conductive treatment (platinum vapor deposition). Thus, a measurement sample was prepared. Next, the measurement sample was observed with an electron microscope (JSM-7800F PRIME, manufactured by JEOL Ltd.) at a magnification of 10,000, and the coordinates of 1,000 inorganic spherical particles in the resultant observation image were determined by analyzing the observation image with image analysis software ("Simple Digitizer ver. 3.2" free software). One pair of coordinates of an inorganic spherical particle was randomly selected from the resultant coordinate data, and the following circle was drawn around the selected inorganic spherical particle serving as a center: such a distance "t" from the center that at least 200 inorganic spherical particles were included in the circle was defined as the radius of the circle. The number of the spherical particles in the circle was determined, and an average particle density $<\rho>$ (unit: particles/cm$^2$) was calculated. dr is a value of from about $r_0/100$ to about $r_0/10$ ($r_0$ represents the average primary particle diameter of the inorganic spherical particles), and the number dn of the particles in a region between a circle distant from the central inorganic spherical particle by "r" and a circle distant therefrom by r+dr, and the area da of the region are determined. The following equation was calculated by using the values of the $<\rho>$, the dn, and the da thus determined.

.

$$g(r) = \{1/<\rho>\} \times \{dn/da\}$$

Thus, the radial distribution function $g(r)$ was determined. After that, a graph showing a relationship between the radial distribution function and the ratio "$r/r_0$" ("$r$" represented an arbitrary distance from the center of the circle, and $r_0$ represented the average primary particle diameter of the inorganic spherical particles) was produced, and whether or not the following conditions (I) and (II) were satisfied was evaluated on the basis of the resultant graph. In Table 5, the symbol "A" means that the condition (I) and the condition (II) are each satisfied, and the symbol "B" means that the condition (I) and the condition (II) are not satisfied respectively.

(I) A nearest-neighbor particle distance $r_1$ defined as the "$r$" corresponding to the peak top of a peak that is closest to an origin out of peaks appearing in the radial distribution function graph is a value once or more and twice or less as large as the average particle diameter $r_0$.

(II) The local minimum value of the radial distribution function $g(r)$ between a second-nearest-neighbor particle distance $r_2$, which is defined as the "$r$" corresponding to the peak top of a peak that is second closest to the origin out of the peaks appearing in the radial distribution function graph, and the nearest-neighbor particle distance $r_1$ is a value of 0.56 or more and 1.10 or less.

Table 4

| | | Composition of curable composition | | | | | | | | | Rare earth metal fluoride content [mass%] |
| | | Monomer composition | | G-PID | | X-ray opaque filler | | | | | |
| | | Abbreviation | Part(s) by mass | Abbreviation | Part(s) by mass | Abbreviation | Configuration | Average particle diameter [$\mu$m] | Full width at half maximum C [°] | Part(s) by mass | |
|---|---|---|---|---|---|---|---|---|---|---|---|
| Reference Example | 1 | M1 | 20 | PF-1 | 64 | CF-1 | F-3/M2 cured product | 24.9 | 0.40 | 16 | 12 |
| | 2 | | | | 64 | CF-2 | F-4/M2 cured product | 24.7 | 0.53 | 16 | 12 |
| | 3 | | | | 64 | CF-3 | F-5/M2 cured product | 26.3 | 0.58 | 16 | 12 |
| | 4 | | | | 64 | CF-4 | F-6/M2 cured product | 25.2 | 0.75 | 16 | 12 |
| | 5 | | | | 64 | CF-1a | F-3/M2 cured product | 4.8 | 0.41 | 16 | 12 |
| | 6 | | | | 64 | CF-1b | | 7.6 | 0.40 | 16 | 12 |
| | 7 | | | | 64 | CF-1c | | 9.4 | 0.42 | 16 | 12 |
| | 8 | | | | 64 | CF-1d | | 10.5 | 0.42 | 16 | 12 |
| | 9 | | | | 64 | CF-1e | | 14.8 | 0.40 | 16 | 12 |
| | 10 | | | | 64 | CF-1f | | 17.5 | 0.41 | 16 | 12 |
| | 11 | | | | 64 | CF-1g | | 54.4 | 0.41 | 16 | 12 |
| | 12 | | | | 64 | CF-1h | | 86.4 | 0.41 | 16 | 12 |
| | 13 | | | | 64 | CF-1i | | 102.3 | 0.42 | 16 | 12 |

| | | Composition of curable composition | | | | | | | | | Rare earth metal fluoride content [mass%] |
|---|---|---|---|---|---|---|---|---|---|---|---|
| | | Monomer composition | | G-PID | | X-ray opaque filler | | | | | |
| | | Abbreviation | Part (s) by mass | Abbreviation | Part (s) by mass | Abbreviation | Configuration | Average particle diameter [μm] | Full width at half maximum C [°] | Part (s) by mass | |
| Reference Comparative Example | 1 | M1 | 20 | PF-1 | 80 | - | - | - | - | - | 0 |
| | 2 | | | | 68 | F-1 | F-1 | 0.6 | 0.17 (*) | 12 | 12 |
| | 3 | | | | 68 | F-3 | F-3 | 0.4 | 0.43 (*) | 12 | 12 |
| | 4 | | | | 68 | F-6 | F-6 | 0.4 | 0.77 (*) | 12 | 12 |
| | 5 | | | | 64 | RCF-1 | F-1/M2 cured product | 24.7 | 0.19 | 16 | 12 |

* The values of Comparative Examples 2 to 4 each mean the full width at half maximum S.

Table 5

| | | X-ray radiography contrast [Al%] | Transparency (Yb/Yw) | Spectral reflectance ratio (SR1/SR2) | Bending strength [MPa] | Evaluation of radial distribution function | |
| --- | --- | --- | --- | --- | --- | --- | --- |
| | | | | | | Condition (I) | Condition (II) |
| Reference Example | 1 | 356 | 0.31 | 1.00 | 134 | A | A |
| | 2 | 352 | 0.30 | 1.02 | 137 | A | A |
| | 3 | 350 | 0.29 | 1.03 | 137 | A | A |
| | 4 | 356 | 0.25 | 1.12 | 140 | A | A |
| | 5 | 290 | 0.31 | 0.99 | 129 | A | A |
| | 6 | 293 | 0.31 | 1.01 | 130 | A | A |
| | 7 | 291 | 0.31 | 1.00 | 135 | A | A |
| | 8 | 309 | 0.31 | 1.00 | 141 | A | A |
| | 9 | 289 | 0.31 | 1.02 | 138 | A | A |
| | 10 | 293 | 0.31 | 1.01 | 139 | A | A |
| | 11 | 421 | 0.31 | 1.02 | 117 | A | A |
| | 12 | 454 | 0.31 | 1.00 | 98 | A | A |
| | 13 | 462 | 0.31 | 1.02 | 93 | A | A |
| Reference Comparative Example | 1 | 180 | 0.30 | 1.16 | 153 | A | A |
| | 2 | 259 | 0.52 | 0.80 | 89 | B | A |
| | 3 | 262 | 0.48 | 0.82 | 87 | B | A |
| | 4 | 276 | 0.30 | 0.85 | 88 | B | A |
| | 5 | 359 | 0.40 | 0.86 | 138 | A | A |

The curable composition of Reference Comparative Example 1 is an example of the base existing structural color-based dental curable composition, and is prepared so as to show a spectral reflectance of 1.16 so that the composition may show excellent color tone compatibility (express a structural color in a yellow to red region) when used as a CR for restoring a cavity formed in dentin or over the range of from enamel to the dentin. However, the composition does not show sufficient X-ray radiography contrast because no X-ray opaque filler is blended thereinto.

As is understood from the results of Reference Comparative Examples 2 to 4, when the crystalline rare earth metal fluoride particles are blended without being subjected to organic-inorganic composition, an improvement in X-ray radiography contrast is observed, but as described above, the crystalline rare earth metal fluoride particles enter a space in the G-PID to inhibit the formation of a periodic structure, and hence the condition (I) in the evaluation of the radial distribution function is not satisfied, with the result that the spectral reflectance ratios are lower (than the value of Comparative Example 1 free of the crystalline rare earth metal fluoride particles).

As is understood from the results of Reference Comparative Example 5, when the crystalline rare earth metal fluoride particles having a full width at half maximum of less than 0.3° are blended after having been subjected to organic-inorganic composition, the X-ray radiography contrast and the bending strength are improved, and the conditions (I) and (II) in the evaluation of the radial distribution function are satisfied, but the cured body becomes more opaque owing to light refraction or scattering between the crystalline rare earth metal fluoride particles and the matrix as compared to that in the case where no crystalline rare earth metal fluoride particles are blended (Comparative Example 1), with the result that its spectral reflectance ratio reduces.

As is understood from the results of Reference Examples 1 to 4, when the crystalline rare earth metal fluoride particles having a full width at half maximum of 0.3° or more are blended after having been subjected to organic-inorganic composition (the specific X-ray opaque filler is blended), improvements in X-ray radiography contrast and bending strength are observed as in Reference Comparative Example 5, and moreover, a reduction in spectral reflectance ratio is suppressed, and hence a relatively high spectral reflectance ratio is maintained.

As is understood from the results of Reference Examples 5 to 13, as the particle diameter of the X-ray opaque filler to be blended becomes smaller, there is observed such a tendency that the X-ray radiography contrast reduces and the bending strength is improved, but the particle diameter of the X-ray opaque filler does not affect the transparency, the spectral reflectance ratio, and the evaluation of the radial distribution function.

4. Examples and Comparative Examples Examples 1 to 10, and Comparative Examples 1 and 2

(1) Secondary Raw Material Particulate Object-preparing Step

The highly crystalline rare earth metal fluoride particulate object YbF3-40 was subjected to wet mechanochemical treatment in conformity with the method described in the subsection "(1) Mechanochemical Treatment of Crystalline Rare Earth Metal Fluoride Particulate Object" of the section "3-1. Production of X-ray Opaque Filler" under conditions shown in Table 6. The resultant slurry was subjected to spray drying with a disc-type spray dryer under the conditions of a slurry concentration of from 30 wt% to 40 wt%, a number of revolutions of from 18,000 rpm to 20,000 rpm, and an inlet temperature of from 200°C to 300°C to provide each of secondary raw material particulate objects SF-1 to SF-5. The average primary particle diameters and full widths at half maximum of the secondary raw material particulate objects thus obtained were measured in the same manner as in Examples for Reference. Table 6 shows the results.

Table 6

| Secondary raw material particulate object | Rare earth metal fluoride particles | Mechanochemical treatment time [min.] | Average primary particle diameter [nm] | Maximum peak: (111) plane | |
|---|---|---|---|---|---|
| | | | | $2\theta$ [°] | Full width at half maximum S [°] |
| SF-1 | F-3 | 180 | 44 | 27.9 | 0.43 |
| SF-2 | F-2 | 90 | 39 | 28.0 | 0.32 |
| SF-3 | F-4 | 240 | 33 | 27.9 | 0.51 |
| SF-4 | F-5 | 300 | 28 | 27.8 | 0.59 |
| SF-5 | F-6 | 600 | 16 | 27.9 | 0.77 |

(2) Curable Raw Material Composition-preparing Step

100 Parts by mass of the monomer composition M1 and the secondary raw material particulate object whose amount ratio was shown in Table 7 were kneaded under conditions shown in Table 7 with a twin-screw planetary type kneading apparatus capable of a vacuum pressure reduction operation under a state in which the internal temperature of the kneading apparatus was kept at from 25°C to 50°C. The result of whether or not the kneading was able to be performed without any problem (kneading feasibility) is also shown in Table 7. In Table 7, the symbol "A" means that the kneading was able to be performed, and the symbol "B" means that the kneading was not able to be performed.

Table 7

| | | Raw material composition | | | | | Kneading conditions | | Kneading feasibility | Average particle diameter after granulation step [μm] |
| | | Monomer | | Secondary raw material particulate object | | Filling ratio [mass%] | Pressure [Pa] | Kneading time [min] | | |
| | | Composition | Part(s) by mass | Abbreviation | Part(s) by mass | | | | | |
|---|---|---|---|---|---|---|---|---|---|---|
| Example | 1 | M1 | 100 | SF-1 | 300 | 75 | $1.0 \times 10^5$ | 400 | A | 24.8 |
| | 2 | ↑ | ↑ | ↑ | ↑ | ↑ | $3.2 \times 10^4$ | 380 | A | 24.5 |
| | 3 | ↑ | ↑ | ↑ | ↑ | ↑ | $1.0 \times 10^4$ | 250 | A | 24.9 |
| | 4 | ↑ | ↑ | ↑ | ↑ | ↑ | $1.0 \times 10^2$ | 180 | A | 24.6 |
| | 5 | ↑ | ↑ | ↑ | ↑ | ↑ | 1.0 | 100 | A | 25.1 |
| | 7 | ↑ | ↑ | ↑ | 233 | 70 | $1.0 \times 10^2$ | 150 | A | 24.8 |
| | 8 | ↑ | ↑ | ↑ | 300 | 75 | $1.0 \times 10^3$ | 200 | A | 25.3 |
| | 9 | ↑ | ↑ | ↑ | 400 | 80 | $1.0 \times 10^3$ | 250 | A | 24.1 |
| | 10 | ↑ | ↑ | ↑ | 900 | 90 | $1.0 \times 10^3$ | 300 | A | 23.2 |
| | 11 | ↑ | ↑ | SF-2 | 300 | 75 | $3.2 \times 10^4$ | 380 | A | 24.8 |
| | 12 | ↑ | ↑ | SF-3 | ↑ | ↑ | ↑ | ↑ | ↑ | 24.3 |
| | 13 | ↑ | ↑ | SF-4 | ↑ | ↑ | ↑ | ↑ | ↑ | 24.1 |
| | 14 | ↑ | ↑ | SF-5 | ↑ | ↑ | ↑ | ↑ | ↑ | 23.8 |
| Comparative Example | 1 | ↑ | ↑ | SF-1 | 300 | 75 | Atmospheric Pressure ($1.01 \times 10^5$) | - | B | - |
| | 2 | ↑ | ↑ | ↑ | ↑ | 75 | $1.0 \times 10^{-1}$ | - | B | - |

EP 4 751 698 A1

36

As shown in Table 7, Comparative Example 1 is an example in which the kneading was performed under atmospheric pressure without any pressure reduction. When the kneading was performed under atmospheric pressure, the polymerizable monomer did not infiltrate the secondary raw material particulate object, and hence the mixture did not become pasty. Accordingly, the curable raw material composition was not able to be kneaded. In addition, as understood from the result of Comparative Example 2, when the degree of vacuum was high, the powder and the polymerizable monomer scattered at the time of the pressure reduction. Accordingly, the curable raw material composition was not able to be kneaded. As understood from the result of Example 1, when the kneading was performed under reduced pressure, the curable raw material composition was able to be kneaded. In addition, as understood from the results of Examples 1 to 5, an increase in pressure facilitated the integration of the curable raw material composition and shortened its kneading time. Further, as understood from the results of Examples 7 to 10, it was found that the kneading was able to be performed under reduced pressure when the amount of the secondary raw material particulate object was from 70 parts by mass to 90 parts by mass with respect to 100 parts by mass of the polymerizable monomer.

(2) Curing Step and Granulation Step

Next, (for each of the systems in which the kneading was able to be performed,) the resultant curable raw material composition was cured with a nitrogen pressure heat polymerizer POLYNER (manufactured by Towa Giken K.K.) to form a bulk material. After that, the bulk material was heated at 100°C for 30 minutes under nitrogen pressure to be cured, to thereby form a bulk material. After that, the resultant cured body and zirconia balls (diameter: 25 mm) were loaded into a zirconia-made pot and subjected to rotational pulverization treatment for 60 minutes to provide a pulverized product of the cured body. Further, a particulate object (X-ray opaque filler) having an average particle diameter shown in Table 7 was obtained by removing coarse particles from the pulverized product with a stainless steel-made sieve having an aperture of 45 μm.

When a curable composition was prepared and evaluated by using the X-ray opaque filler thus obtained in the same manner as in Examples for Reference, results similar to those of Examples for Reference were obtained.

**Claims**

**1.** A method of producing an X-ray opaque filler, which is configured to impart X-ray opacity to each of a dental curable composition containing a polymerizable monomer and a cured body thereof by being blended into the dental curable composition, wherein

when such a particulate object formed of crystalline rare earth metal fluoride particles that a full width at half maximum of a maximum intensity peak in an X-ray diffraction pattern obtained when the particulate object is subjected to X-ray diffraction measurement is less than 0.3° is defined as "highly crystalline rare earth metal fluoride particulate object," and such a particulate object that the full width at half maximum is 0.3° or more is defined as "low crystalline rare earth metal fluoride particulate object,"
the X-ray opaque filler contains, as a main component, a particulate object, which includes organic-inorganic composite particles each formed of a composite material obtained by dispersing the low crystalline rare earth metal fluoride particulate object in a resin matrix, and which has an average particle diameter measured by a laser diffraction/scattering method of from 3 μm to 110 μm,
the method comprising:

a secondary raw material particulate object-preparing step of subjecting a raw material slurry, which is obtained by dispersing a primary raw material particulate object formed of a highly crystalline rare earth metal fluoride particulate object having an average primary particle diameter measured by electron microscope observation of from 1 nm to 500 nm in a dispersion medium, to mechanochemical treatment to provide a treated slurry liquid in which the low crystalline rare earth metal fluoride particulate object is dispersed in the dispersion medium, followed by spray drying of the treated slurry thus obtained to provide a secondary raw material particulate object formed of the low crystalline rare earth metal fluoride particulate object;
a curable raw material composition-preparing step of kneading 100 parts by mass of the polymerizable monomer serving as a raw material for the resin matrix and 150 parts by mass to 900 parts by mass of the secondary raw material particulate object under a pressure of from 1.0 to $1.0 \times 10^5$ (Pa) to provide a curable raw material composition;
a curing step of curing the curable raw material composition to provide a bulk material formed of a composite material obtained by dispersing low crystalline rare earth metal fluoride particles in the resin matrix; and
a granulation step of pulverizing the bulk material, followed by particle size adjustment so that the average particle diameter becomes from 3 μm to 110 μm.

2. The method of producing an X-ray opaque filler according to claim 1, wherein an amount of the secondary raw material particulate object to be used in the curable raw material composition-preparing step is from 230 parts by mass to 900 parts by mass with respect to 100 parts by mass of the polymerizable monomer.

3. The method of producing an X-ray opaque filler according to claim 1, wherein the kneading in the curable raw material composition-preparing step is performed at from 100 to 35,000 (Pa).

4. A method of producing a dental curable composition, which is capable of providing a cured body that expresses a structural color having a predetermined color tone independent of an incident angle of light and has X-ray radiography contrast, by mixing:

   100 parts by mass of a polymerizable monomer;
   1 part by mass to 100 parts by mass of an X-ray opaque filler containing, as a main component, a particulate object, which includes organic-inorganic composite particles each formed of a composite material obtained by dispersing a low crystalline rare earth metal fluoride particulate object in a resin matrix, and which has an average particle diameter measured by a laser diffraction/scattering method of from 3 μm to 110 μm, in terms of a total mass of the low crystalline rare earth metal fluoride particulate object;
   10 parts by mass to 1,500 parts by mass in total of one or a plurality of "groups of spherical particles having identical diameters," which are each formed of an aggregate of inorganic spherical particles having a predetermined average primary particle diameter measured by electron microscope observation in a range of from 100 nm to 1,000 nm, the individual inorganic spherical particles for forming the aggregate including substantially the same substance, and 90% or more of a total number of the particles being present in a range of ±5% of the predetermined average primary particle diameter in a number-based particle size distribution of the aggregate; and
   0.01 part by mass to 10 parts by mass of a polymerization initiator,
   the method further comprising, as a step of preparing the X-ray opaque filler, a step of producing the X-ray opaque filler by the production method of claim 1,
   wherein in the step, the X-ray opaque filler is produced by using a polymerizable monomer serving as a raw material for the resin matrix, the polymerizable monomer satisfying the following condition: when a refractive index of a cured body of the polymerizable monomer for a sodium d-line at 25°C is represented by $n_{(MX)}$, and a refractive index of a resin material for forming the resin matrix of each of the organic-inorganic composite particles for forming the X-ray opaque filler for the sodium d-line at refractive index 25°C is represented by $n_{(F-MX)}$, an absolute value $|n_{(MX)}-n_{(F-MX)}|$ of a difference between the $n_{(MX)}$ of the polymerizable monomer to be actually mixed and the $n_{(F-MX)}$ is from 0 to 0.1.

5. The method of producing a dental curable composition according to claim 4, wherein

   when the number of the groups of spherical particles having identical diameters" in the one or the plurality of the "groups of spherical particles having identical diameters" is represented by "a", the respective "groups of spherical particles having identical diameters" are each represented by G-PID$_m$ in order of increasing average primary particle diameter, provided that "m" represents 1 when "a" represents 1, and "m" represents a natural number of from 1 to "a" when "a" represents 2 or more, and a refractive index of the inorganic spherical particles for forming each of the G-PID$_m$s for the sodium d-line at 25°C is represented by $n_{(G-PIDm)}$,
   the substances for forming the individual particles of the respective G-PID$_m$s in a case where the "a" represents 2 or more may be different from each other,
   the average primary particle diameters of the respective G-PID$_m$s in the case are different from each other by 25 nm or more, and
   a relationship of $n_{(MX)}<n_{(G-PIDm)}$ is valid for any one of the $n_{(G-PIDm)}$s.

## INTERNATIONAL SEARCH REPORT

| International application No. |
|---|
| **PCT/JP2024/019347** |

### A. CLASSIFICATION OF SUBJECT MATTER

*A61K 6/887*(2020.01)i; *A61K 6/15*(2020.01)i; *A61K 6/16*(2020.01)i; *A61K 6/17*(2020.01)i; *C08F 220/20*(2006.01)i;
*C08F 220/26*(2006.01)i; *C08F 220/36*(2006.01)i
FI:   A61K6/887; A61K6/15; A61K6/16; A61K6/17; C08F220/20; C08F220/26; C08F220/36

According to International Patent Classification (IPC) or to both national classification and IPC

### B. FIELDS SEARCHED

Minimum documentation searched (classification system followed by classification symbols)

A61K6/887; A61K6/15; A61K6/16; A61K6/17; C08F220/20; C08F220/26; C08F220/36

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| A | WO 2020/050123 A1 (TOKUYAMA DENTAL CORP.) 12 March 2020 (2020-03-12) claims, examples, paragraphs [0096]-[0103], [0109] | 1-5 |
| A | WO 2021/131490 A1 (TOKUYAMA DENTAL CORP.) 01 July 2021 (2021-07-01) claims, examples, paragraphs [0082]-[0089] | 1-5 |
| A | WO 2023/042598 A1 (TOKUYAMA DENTAL CORP.) 23 March 2023 (2023-03-23) claims, examples | 1-5 |
| A | WO 2023/085201 A1 (TOKUYAMA DENTAL CORP.) 19 May 2023 (2023-05-19) claims, examples, paragraphs [0033]-[0035] | 1-5 |
| A | JP 2022-184770 A (TOKUYAMA DENTAL CORP.) 13 December 2022 (2022-12-13) claims, examples, production example 14, example 12 | 1-5 |

☐ Further documents are listed in the continuation of Box C.      ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **02 August 2024** | **13 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

### INTERNATIONAL SEARCH REPORT
#### Information on patent family members

International application No.

**PCT/JP2024/019347**

| Patent document cited in search report | | | Publication date (day/month/year) | Patent family member(s) | | | Publication date (day/month/year) |
|---|---|---|---|---|---|---|---|
| WO | 2020/050123 | A1 | 12 March 2020 | US | 2021/0324178 | A1 | |
| | | | | claims, examples, paragraphs [0106]-[0113], [0119] | | | |
| | | | | JP | 2020-37534 | A | |
| | | | | JP | 2020-90574 | A | |
| | | | | EP | 3848418 | A1 | |
| | | | | CN | 112639017 | A | |
| | | | | KR | 10-2021-0057026 | A | |
| WO | 2021/131490 | A1 | 01 July 2021 | US | 2023/0045524 | A1 | |
| | | | | claims, examples, paragraphs [0106]-[0118] | | | |
| | | | | EP | 4082516 | A1 | |
| | | | | CN | 114845684 | A | |
| | | | | KR | 10-2022-0120635 | A | |
| WO | 2023/042598 | A1 | 23 March 2023 | CA | 3227331 | A1 | |
| | | | | claims, examples | | | |
| | | | | CN | 117642143 | A | |
| WO | 2023/085201 | A1 | 19 May 2023 | (Family: none) | | | |
| JP | 2022-184770 | A | 13 December 2022 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017069274 A1
- WO 2020050123 A1
- JP 3017803 B
- WO 2023042598 A1
- JP 2023029645 A
- JP 2022031237 W